# EUROPEAN PATENT APPLICATION

(11) **EP 4 389 070 A2**
(43) Date of publication of application: **26.06.2024**
(21) Application number: 23219157.7
(22) Date of filing: 21.12.2023
(51) Int. Cl.: A61F 2/16

(54) **INTRAOCULAR LENS AND INTRAOCULAR LENS INSERTION INSTRUMENT**

(30) Priority: 24.12.2022 JP 2022207694; 31.03.2023 JP 2023058514; 31.03.2023 JP 2023058515
(71) Applicant: Nidek Co., Ltd., Gamagori-shi, Aichi, 443-0038 (JP)
(72) Inventor: NAGASAKA, Shinji, Gamagori-shi Aichi, 443-0038 (JP); MURASE, Kyoko, Gamagori-shi Aichi, 443-0038 (JP); NAKAHATA, Yoshihiro, Gamagori-shi Aichi, 443-0038 (JP); TOBITA, Natsuko, Gamagori-shi Aichi, 443-0038 (JP)
(74) Representative: Viering, Jentschura & Partner mbB Patent- und Rechtsanwälte

(57) **Abstract**

An intraocular lens (1) includes a lens portion (2) having a disc-shape. A cross-section of the lens portion (2) defined by an arbitrary direction that passes through a geometric center O of the lens portion (2) and is perpendicular to a lens surface of the lens portion (2) is defined as a virtual cross-section. A cross-sectional area of the lens portion (2) in the virtual cross-section changes depending on the arbitrary direction that defines the virtual cross-section. When the intraocular lens (1) is folded by the nozzle, a cross-sectional area of the lens portion (2) in a first cross-section, which is the virtual cross-section defined by a direction perpendicular to the pushing axis, is smaller than or equal to a cross-sectional area in the virtual cross-section that is defined by another direction.

## Description

### TECHNICAL FIELD

The present disclosure relates to an intraocular lens that is folded and inserted into an eye by an intraocular lens insertion instrument, and the intraocular lens insertion instrument filled with the intraocular lens.

### BACKGROUND

As one of surgical methods for cataracts, there has been a method where a bendable, soft intraocular lens is inserted into an eye after the crystalline lens is removed. Furthermore, in order to correct a refractive power of an eye, an intraocular lens may be inserted into an area in front of the crystalline lens. An intraocular lens insertion instrument called an injector is sometimes used to insert the intraocular lens into an eye.

For example, in the intraocular lens insertion instrument described in JP 2016-190023 A, the entire intraocular lens is folded into a small size by being pushed forward by a pushing member. Thereafter, the intraocular lens is inserted into the eye from the tip of a nozzle of the intraocular lens insertion instrument that is inserted into the eye through an incision.

The intraocular lens insertion instrument may fold the intraocular lens by deforming supporters of the intraocular lens and positioning the supporters on a lens portion (i.e., an optical part) (so-called a "tacking" state). By performing tacking, the possibility of damage to the supporters may decrease. In the intraocular lens insertion instrument described in JP 2016-019608 A, a rod-shaped pushing member moves forward along a pushing axis. Accordingly, the intraocular lens is inserted into the eye after passing through a passage in the nozzle while a rear supporter extending rearward in the pushing axis is being tacked.

### SUMMARY

If load applied when the intraocular lens in the nozzle passage is folded and pushed out increases, the difficulty of the surgery would increase or the possibility of damage to the intraocular lens would increase. If the intraocular lens is folded into a smaller size within the nozzle passage, the load applied when the intraocular lens is pushed out through the nozzle passage may be reduced. In addition, when inserting an intraocular lens into a patient's eye, the incision for inserting the nozzle of the intraocular lens insertion instrument into the eye should be made as small as possible to reduce the burden on the patient. The smaller the nozzle diameter is, the easier the nozzle is inserted into the eye through a small incision. Here, if the intraocular lens can be folded into a small size within the passage of the nozzle, is also easier to reduce the diameter of the nozzle. In this way, a technique for folding an intraocular lens into a smaller size within the nozzle passage of an intraocular lens insertion instrument has been desired.

A typical objective of this disclosure is to provide an intraocular lens that can be folded into a small size within a nozzle passage of an intraocular lens insertion instrument, and the intraocular lens insertion instrument filled with the intraocular lens.

A first aspect of the present disclosure is an intraocular lens that is deformable and is inserted into a patient's eye from an insertion port formed at a front end of an intraocular lens insertion instrument after passing through an internal passage of the intraocular lens insertion instrument. The intraocular lens insertion instrument includes: a pushing member that is configured to push the intraocular lens forward by moving forward in the internal passage along a pushing axis that is an axis of the internal passage; and a nozzle that includes the insertion port at the front end and defines therein the internal passage that is tapered toward a front side of the nozzle, the intraocular lens being discharged from the nozzle through the insertion port after the intraocular lens was folded while being pushed forward in the internal passage by the pushing member. The intraocular lens includes a lens portion having a disc-shape. A cross-section of the lens portion defined by an arbitrary direction that passes through a geometric center of the lens portion and is perpendicular to a lens surface of the lens portion is defined as a virtual cross-section. A cross-sectional area of the lens portion in the virtual cross-section changes depending on the arbitrary direction that defines the virtual cross-section. When the intraocular lens is folded by the nozzle, a cross-sectional area of the lens portion in a first cross-section, which is the virtual cross-section defined by a direction perpendicular to the pushing axis, is smaller than or equal to a cross-sectional area in the virtual cross-section that is defined by another direction.

A second aspect of the present disclosure is an intraocular lens insertion instrument that is configured to insert an intraocular lens, which has been filled in advance and is deformable, into a patient's eye through an insertion port formed at a front end of the intraocular lens insertion instrument after passing through an internal passage of the intraocular lens insertion instrument. The instrument includes: a pushing member that is configured to push the intraocular lens forward by moving forward in the internal passage along a pushing axis that is an axis of the internal passage; and a nozzle that includes the insertion port at the front end and defines therein the internal passage that is tapered toward a front side of the nozzle, the intraocular lens being discharged from the nozzle through the insertion port after the intraocular lens was folded while being pushed forward in the internal passage by the pushing member. The intraocular lens includes a lens portion having a disc-shape. A cross-section of the lens portion defined by an arbitrary direction that passes through a geometric center of the lens portion and is perpendicular to a lens surface of the lens portion is defined as a virtual cross-section. A cross-sectional area of the lens portion in the virtual cross-section changes depending on the arbitrary direction that defines the virtual cross-section. When the intraocular lens is folded by the nozzle, a cross-sectional area of the lens portion in a first cross-section, which is the virtual cross-section defined by a direction perpendicular to the pushing axis, is smaller than or equal to a cross-sectional area in the virtual cross-section defined by another direction.

A third aspect of the present disclosure is an intraocular lens that is deformable and is inserted into a patient's eye from an insertion port formed at a front end of an intraocular lens insertion instrument after passing through an internal passage of the intraocular lens insertion instrument. The intraocular lens includes: a lens portion that has a disc-shape and one or more supporters that outwardly extend from the lens portion to have a curved shape. The intraocular lens insertion instrument includes: a pushing member that is configured to push out, by moving forward in the internal passage along a pushing axis that is an axis of the internal passage, the intraocular lens forward while a rear supporter, which is the one or more supporters extending rearward from the lens portion, is being folded onto the lens portion by the pushing member in contact with the rear supporter; and a nozzle that includes the insertion port at the front end and defines therein the internal passage that is tapered toward a front side of the nozzle, the intraocular lens being discharged from the nozzle through the insertion port after the intraocular lens was folded while being pushed forward in the internal passage by the pushing member. A cross-section that is perpendicular to a lens surface of the lens portion and extends in an arbitrary direction outward from the geometric center of the lens portion is defined as a virtual partial cross-section. A cross-sectional area of the lens portion in the virtual partial cross-section changes depending on the arbitrary direction that defines the virtual partial cross-section. When the intraocular lens is folded by the nozzle, a cross-sectional area of an on-axis rear cross-section, which is the virtual partial cross-section extending rearward from the geometric center along the pushing axis, is smaller than or equal to a cross-sectional area of the virtual partial cross-section defined by another direction.

A fourth aspect of the present disclosure is an intraocular lens insertion instrument that is configured to insert an intraocular lens, which has been filled in advance and is deformable, into a patient's eye through an insertion port formed at a front end of the intraocular lens insertion instrument after passing through an internal passage of the intraocular lens insertion instrument. The intraocular lens includes a lens portion having a disc-shape and one or more supporters that outwardly extend from the lens portion to have a curved shape. The instrument includes: a pushing member that is configured to push out, by moving forward in the internal passage along a pushing axis that is an axis of the internal passage, the intraocular lens forward while a rear supporter, which is the one or more supporters extending rearward from the lens portion, is being folded onto the lens portion by the pushing member in contact the rear supporter; and a nozzle that includes the insertion port at the front end and defines therein the internal passage that is tapered toward a front side of the nozzle, the intraocular lens being discharged from the nozzle through the insertion port after the intraocular lens was folded while being pushed forward in the internal passage by the pushing member. A cross-section that is perpendicular to a lens surface of the lens portion and extends in an arbitrary direction outward from the geometric center of the lens portion is defined as a virtual partial cross-section. A cross-sectional area of the lens portion in the virtual partial cross-section changes depending on the arbitrary direction that defines the virtual partial cross-section. When the intraocular lens is folded by the nozzle, a cross-sectional area of an on-axis rear cross-section, which is the virtual partial cross-section extending rearward from the geometric center along the pushing axis, is smaller than or equal to a cross-sectional area of the virtual partial cross-section defined by another direction.

According to the intraocular lens and the intraocular lens insertion instrument according to the present disclosure, the intraocular lens is easily folded into a small size within the nozzle passage of the intraocular lens insertion instrument.

### <Another aspect>

Next, another aspect of the intraocular lens in the present disclosure will be described. The intraocular lens exemplified in the present disclosure includes a disc-shaped lens portion. Three or more segmented regions having mutually different refractive powers are formed on at least one of a front surface and a rear surface of the lens portion. The plurality of segmented regions include a distant vision region having the smallest refractive power, a near vision region having the largest refractive power, and an intermediate vision region having a refractive power between the refractive power of the distant vision region and the refractive power of the near vision region. The plurality of segmented regions extend radially outward from a center of the lens portion, and have different radii of curvature and thus have the different refractive powers.

In the intraocular lens exemplified in the present disclosure, the plurality of segmented regions having different refractive powers extend radially outward from the center of the lens portion. Therefore, unlike an intraocular lens in which a plurality of regions are arranged concentrically, light easily passes through all the plurality of segmented regions even when the wearer's pupil becomes smaller. Furthermore, the plurality of segmented regions have different radii of curvature and thus have different refractive powers. Therefore, the amount of light scattered in unintended directions is reduced as compared to a situation where a group of micro prisms or the like is formed in the segmented region. As a result, loss of light reaching the retina is less likely to occur, making it easier to obtain a better visual field. In addition, it is easy to process the intraocular lens with high precision and is less likely to be damaged when inserted into an eye.

The plurality of segmented regions may extend radially outward from one reference point in the center of the lens portion. In other words, in each segmented region, linear ends extending outward from the center of the lens portion may all pass through the same reference point. In this case, even if the wearer's pupil becomes smaller, the amount of light passing through each of the plurality of segmented regions can be easily secured as compared with a situation where a fixed region (for example, a circular region) is separately formed at the center of the lens portion. As a result, a multifocal effect can be easier obtained regardless of the size of the wearer's pupil, so the wearer's field of vision becomes easier to improve.

Note that the lens surface of each segmented region may be a spherical surface or an aspherical surface. Furthermore, the refractive power is approximately constant within each segmented region. In other words, when a transition portion, which will be described later, is formed on the lens surface, the refractive power changes within the transition region, whereas the refractive power is approximately constant within each segmented region.

The transition portion may be formed between the plurality of segmented regions in the lens portion. The transition portion may a radius of curvature that continuously changes from the end of one of a pair of segmented regions adjacent to each other via the transition portion to the end of the other one of the pair of segmented regions. As a result, the ends of the pair of segmented regions may be smoothly connected to each other. If the transition portion is not formed in the lens portion, a step or the like may be formed at the boundary between a pair of adjacent segmented regions, and such a step would cause a phenomenon in which light is diffusely reflected by the step or the like, deteriorating the visual field (for example, a halo or glare, etc.). On the contrary, by forming the transition portion between a pair of adjacent segmented regions, the influence of diffused reflection of light due to a step or the like is less likely to occur. Furthermore, when the transition portion is formed in the lens portion, the refractive power of each region within the transition portion smoothly shifts from the refractive power of one of the adjacent segmented regions to the refractive power of the other of the segmented regions. As a result, in addition to the refractive power of each segmental region, a refractive power between the refractive powers of the segmented regions is also applied to the lens portion. Therefore, by forming the transition portions in the lens portion, the effect of expanded depth of focus (EDOF) of the multifocal intraocular lens can also be appropriately obtained.

Both the segmented region and the transition portion may refract light, in a direction toward the optical axis, that is incident on the lens portion in parallel with the optical axis of the lens portion. In this case, the amount of light that is lost without reaching the retina after passing through the lens portion is reduced. Therefore, it becomes easier to obtain a better field of view. Note that it is also possible to manufacture the intraocular lens without forming a transition portion in the lens portion. Even in this case, it is easier to obtain a good visual field as compared to a situation where a group of micro prisms or the like are formed in the segmented region.

The central angle of the transition portion extending outward from the central portion of the lens portion may be greater than or equal to 5 degrees and less than or equal to 30 degrees. In this case, both the multifocal effect by the plurality of segmented regions and the depth of focus expansion effect can be appropriately obtained.

Note that the central angle of the transition portion may be greater than or equal to 15 degrees and less than or equal to 20 degrees. In this case, both the multifocal effect by the plurality of segmented regions and the depth of focus expansion effect can be appropriately obtained.

For the distant vision region, the near vision region, and the intermediate vision region, which spread outward from the center of the lens portion, the central angle of the distant vision region may be largest, and the central angle of the intermediate vision region may be smallest. In this case, distant vision using the distant vision region and near vision using the near vision region can be appropriately obtained with expanded depth of focus.

The central angle of the distant vision region may be 140 degrees or more, the central angle of the near vision region may be 90 degrees or more, and the central angle of the intermediate vision region may be 30 degrees or more. In this case, distant vision using the distant vision region and near vision using the near vision region can be appropriately obtained with expanded depth of focus.

The refractive power in the near vision region may be +2.5D or more and +4.0D or less, and the refractive power in the intermediate vision region may be +1.0D or more and +2.5D or less. In this case, both the multifocal effect by the plurality of segmented regions and the depth of focus expansion effect can be appropriately obtained.

The refractive power in the near vision region may be +3.0D or more and +3.5D or less, and the refractive power in the intermediate vision region may be +1.5D or more and +2.0D or less. In this case, both the multifocal effect and the depth of focus expansion effect can be appropriately obtained.

A toric surface may be formed on at least one of the front and rear surfaces of the lens portion to correct astigmatism of the wearer. In this case, the intraocular lens not only provides both distant vision and near vision, but also corrects astigmatism of the wearer.

Note that the plurality of segmented regions may be formed on the front surface of the lens surface (that is, the surface facing the front side of the eye (a cornea side) when the lens is placed in the wearer's eye). In this case, the shape of the rear surface of the lens surface tends to be smooth so that it is easier to prevent secondary cataract due to cells etc. entering between the rear surface of the lens surface and the posterior capsule of the eye from occurring. Furthermore, a plurality of segmented regions and the toric surface may be formed together on the front surface of the lens surface. In this case, even when the toric surface is formed on the lens surface, occurrence of secondary cataract can be easily prevented. However, it is also possible to form at least one of the segmented region and the toric surface on the rear surface of the lens surface. The surface on which the segmented region is formed and the surface on which the toric surface is formed may be different.

### <First aspect>

A first aspect of an intraocular lens according to the present disclosure will be described. The intraocular lens exemplified in the present disclosure is deformable and inserted into an eye of a patient from an insertion port at a front end of the intraocular lens through an internal passage of an intraocular lens insertion instrument. The intraocular lens insertion instrument includes a pushing member and a nozzle. The pushing member pushes the intraocular lens forward by moving forward in the passage along a pushing axis that is an axis of the passage. The passage area of the nozzle is formed to decrease (i.e., tapered) toward the front side. The nozzle has the insertion port at its front end, and after folding the intraocular lens while the pushing member pushes the intraocular lens forward through the passage, the intraocular lens is discharged through the insertion port. The intraocular lens includes a substantially disc-shaped lens portion. A cross-section in an arbitrary direction that passes through a geometric center of the lens portion and is perpendicular to the lens surface of the lens portion is defined as a virtual cross-section. The intraocular lens in the present disclosure, the cross-sectional area of the lens portion in the virtual cross-section changes depending on the direction in which the virtual cross-section is defined. When the intraocular lens is folded by the nozzle, the cross-sectional area of the lens portion in a virtual cross-section (a first cross-section) in a direction perpendicular to the pushing axis is less than or equal to cross-sectional areas in virtual cross-sections defined by other directions.

When the intraocular lens in the present disclosure is moved out through the passage of the nozzle at an appropriate angle with respect to the pushing axis of the intraocular lens insertion instrument, the maximum cross-sectional area of the lens portion in the direction perpendicular to the pushing axis (i.e., the cross-sectional area of the first cross-section when the cross-sectional area of the lens portion in a folded state has the maximum value) decreases. Therefore, the intraocular lens in the present disclosure can be folded into a small size within the passage of the nozzle.

The total value of the refractive power on a line extending from the geometric center along the virtual cross-section to one outer circumference and the refractive power on a line extending from the geometric center along the virtual cross-section to the other outer circumference is calculated for each of virtual cross-sections defined by arbitrary directions. In this case, the intraocular lens may be designed such that the total value of the refractive powers on a pair of lines along the first cross-section is equal to or greater than the total value of the refractive powers on a pair of lines along a virtual cross-section defined by another direction.

In the lens portion, the radius of curvature of the portion with high refractive power is smaller than the radius of curvature of the portion with low refractive power. In other words, in the lens portion, the curve of the lens surface in the portion where the refractive power is large is steeper than the curve of the lens surface in the portion where the refractive power is small. Here, since it is optically undesirable to for a step at the geometric center of the lens portion, the thickness of the lens portion at the geometric center in the virtual cross-section is set to be the same regardless of the direction of the assumed virtual cross-section. As a result, the thickness of the side surface (an edge portion) of the lens portion in an area where the refractive power is large (i.e., an area where the curve of the lens surface is steep) is smaller than that of the lens portion where the refractive power is small (i.e., an area where the curve of the lens surface is gentle). Therefore, the cross-sectional area of the lens portion in the virtual cross-section decreases as the total value of the refractive powers on lines extending from the geometric center in both directions along the virtual cross-section increases. Therefore, by designing the intraocular lens to have the total value of the refractive powers on the line along the first cross-section greater than or equal to the total value of the refractive powers on the line along a virtual cross-section in another direction, the cross-sectional area of the lens portion in the first cross-section can be appropriately reduced. As a result, the intraocular lens can be folded into a smaller size within the nozzle passage.

Note that it is also possible to change the configuration of the lens portion in the first cross-section. For example, the total value of the radius of curvature of the lens portion on a line extending from the geometric center to one outer circumference along the virtual cross-section (for example, the average value of the radius of curvature on the line, etc.) and the radius of curvature of the lens portion on a line extending from the geometric center to another circumference along the same virtual cross-section may be calculated for the virtual-cross-section in each arbitrary direction. In this case, the intraocular lens may be designed such that the total value of the radii of curvature on the two lines along the first cross-section is equal to or smaller than the total value of the radii of curvature on a line along a cross-section in another direction. Even in this case, the cross-sectional area of the lens portion in the first cross-section can be appropriately reduced.

Further, when the intraocular lens is a toric intraocular lens that corrects astigmatism of a wearer, a strong principal meridian and a weak principal meridian exist in the lens portion. The strong principal meridian refers to a meridian among arbitrary meridians that has a small radius of curvature and has the maximum refractive power. In addition, the weak principal meridian refers to a meridian with a large radius of curvature and the minimum refractive power. The toric intraocular lens may be designed such that the position of the first cross-section is aligned with the strong principal meridian of the lens portion. Also in this case, the cross-sectional area of the lens portion in the first cross-section is appropriately reduced.

The intraocular lens may further include a pair of supporters. The pair of supporters extend outward from different parts of the side surface of the lens portion and support the lens portion within the patient's eye. The intraocular lens may be designed such that the cross-sectional area of the lens portion in a virtual cross-section (a second cross-section) in a direction passing through the proximal end of each of the pair of supporters is greater than or equal to the cross-sectional area in a virtual cross-section in another direction.

It is preferable that the base end portion of each of the pair of supporters be connected to a thicker portion of the side surface of the lens portion in order to stably support the lens portion within the eye. Furthermore, if the proximal end portion of the supporter is connected to the thinner portion of the side surface of the lens portion, unintended deformation or damage to the proximal end portion of the supporter and the lens portion would occur when the intraocular lens is inserted into the eye and the supporter is bent. The intraocular lens is designed such that the cross-sectional area of the lens portion in the second cross-section in the direction passing through the proximal end portion of each of the pair of supporters is greater than or equal to the cross-sectional area in a virtual cross-section in another direction. Accordingly, the thickness of a portion of the side surface of the lens portion to which the proximal end portions of the pair of supporters are connected increases. As a result, since the rigidity of the proximal end portion of the support portion is secured, unintended deformation or breakage is less likely to occur, and thus the intraocular lens is more easily installed in the eye. Further, since the amount of deformation of the pair of supporters is equalized, the stability during inserting of the intraocular lens into the eye is improved.

The total value of the refractive power on a line extending from the geometric center along the virtual cross-section to one outer circumference and the refractive power on a line extending from the geometric center along the virtual cross-section to the other outer circumference is calculated for each of virtual cross-sections defined in arbitrary directions. In this case, the intraocular lens may be designed such that the total value of the refractive powers on a line along the second cross-section is equal to or smaller than the total value of the refractive powers on a line along a virtual cross-section in another direction.

As described above, the thickness of the side surface (an edge portion) of the lens portion in an area where the refractive power is small (i.e., an area where the curve of the lens surface is gentle) is greater than that of the lens portion where the refractive power is large (i.e., an area where the curve of the lens surface is steep). Therefore, by designing the intraocular lens to have the total value of the refractive power on the line along the second cross-section is greater than or equal to the total value of the refractive power on the line along a virtual cross-section in another direction, the thickness of the portion of the side surface of the lens portion where the proximal end portions of the pair of supporters are connected increases.

Note that it is also possible to change the direction of the second cross-section. For example, the total value of the radius of curvature of the lens portion on a line extending from the geometric center to one outer circumference along the virtual cross-section (for example, the average value of the radius of curvature on the line, etc.) and the radius of curvature of the lens portion on a line extending from the geometric center to another circumference along the same virtual cross-section may be calculated for the virtual-cross-section in each arbitrary direction. In this case, the intraocular lens may be designed such that the total value of the radii of curvature on a pair of lines along the second cross-section is equal to or smaller than the total value of the radii of curvature on a pair of lines along a cross-section in another direction. Even in this case, the thicknesses of the portion of the side surface of the lens portion to which the proximal end portions of the pair of supporters are connected increases.

Further, the toric intraocular lens may be designed such that the position of the second cross-section is aligned with the weak principal meridian of the lens portion. Even in this case, the thicknesses of the portion of the side surface of the lens portion to which the proximal end portions of the pair of supporters are connected increases.

A plurality of segmented regions may be formed on at least one of the front surface and the rear surface of the lens portion. The plurality of segmented regions may extend radially outward from a center of the lens portion, and have different radii of curvature and thus have different refractive powers. The plurality of segmented regions may be arranged in the lens portion such that the cross-sectional area of the lens portion in the first cross-section is equal to or less than a cross-sectional area in a virtual cross-section in another direction.

In this case, unlike an intraocular lens in which a plurality of regions are concentrically arranged, even if the wearer's pupil becomes smaller, light passes through each of the plurality of segmented regions and is focused on the retina. Furthermore, the plurality of segmented regions have different radii of curvature and thus have different refractive powers. Therefore, the amount of light scattered in unintended directions is reduced as compared to a situation where a group of micro prisms or the like is formed in the segmented region. As a result, loss of light reaching the retina is less likely to occur, making it easier to obtain a better visual field. In addition, it is easy to process the intraocular lens with high precision and is less likely to be damaged when inserted into an eye. Further, the intraocular lens is folded into a small size within the passage of the nozzle and inserted into the eye.

However, as described above, the technique illustrated in the second embodiment may also be applied to an intraocular lens in which a plurality of segmented regions are not formed (for example, a toric intraocular lens). Further, both the plurality of segmented regions and a toric surface for correcting astigmatism in the wearer may be formed in the lens portion. Note that among the front and rear surfaces of the lens surface, the surface on which the segmented region is formed and the surface on which the toric surface is formed may be the same surface or may be different surfaces.

The intraocular lens may further include one or more supporters extending outwardly from the lens portion to have a curved shape. By moving forward in the passage along the pushing axis, the pushing member may contact a rear supporter that is the supporter extending rearward from the lens portion and may push out the intraocular lens forward while folding the rear supporter onto the lens portion. When a virtual cross-section in a direction along the pushing axis is defined as a third cross-section, the cross-sectional area of an on-axis rear cross-section of the third cross-section that extends backward from the geometric center along the pushing axis may be smaller than the cross-sectional area of an on-axis front cross-section that extends forward along the pushing axis from the geometric center.

The intraocular lens insertion instrument may fold the intraocular lens by deforming supporters of the intraocular lens and positioning the supporters on a lens portion (so-called a "tacking" state). As a result of repeated trials, the inventor of the present disclosure found that when an intraocular lens with the rear supporter being tacked was pushed out inside the nozzle by the pushing member, load increased when the vicinity of the proximal end portion of the rear supporter passed through the nozzle. This is because the volume of the pushing member that is pushing out the intraocular tens while being in contact with the the rear supporter is added to the increased volume of the vicinity of the proximal end portion due to the bent of the proximal end portion of the rear supporter. As a result, it would be difficult to bend the vicinity of the proximal end portion of the rear supporter into a small size.

On the other hand, by making the cross-sectional area of the on-axis rear section extending rearward from the geometric center along the pushing axis smaller than the cross-sectional area of the on-axis front cross-section extending forward from the geometric center along the pushing axis, an increase in volume of the proximal end portion of the rear supporter that is close to the pushing member is prohibited. As a result, the intraocular lens is easily folded into a small size near the proximal end of the rear supporter where the pushing member is located. Therefore, in addition to the load generated when the geometric center of the lens portion passes through the nozzle, the load generated when the vicinity of the proximal end portion of the rear supporter passes through the nozzle is also appropriately reduced. Therefore, when the intraocular lens is pushed out in the passage of the nozzle by the pushing member, the intraocular lens can be more appropriately folded.

In the present disclosure, the intraocular lens having two loop-shaped (curved) supporters each having a free end is exemplified. However, even in an intraocular lens having three or more loop-shaped supporters, an increase in volume of the vicinity of the proximal end portion of the rear supporter can be prevented by making the cross-sectional area of the on-axis rear cross-section smaller than the cross-sectional area of the on-axis front cross-section,

In the lens portion, the larger the refractive power is (that is, the smaller the radius of curvature is), the smaller the cross-sectional area is. Here, a virtual partial cross-section that is perpendicular to the lens surface of the lens portion and extends in an arbitrary direction outward from the geometric center of the lens portion is assumed. In this case, the cross-sectional area of the lens portion in the virtual partial cross-section decreases as the refractive power in the virtual partial cross-section increases (that is, the radius of curvature decreases). Therefore, the intraocular lens may be designed such that the refractive power of the lens portion in the on-axis rear cross-section is greater than the refractive power of the lens portion in the on-axis front cross-section. In other words, the intraocular lens may be designed such that the radius of curvature of the lens portion in the on-axis rear cross-section is smaller than the radius of curvature of the lens portion in the on-axis front cross-section. In this case, the intraocular lens is easily folded into a small size near the proximal end of the rear supporter where the pushing member is located.

A plurality of segmented regions may be formed on at least one of the front surface and the rear surface of the lens portion. The plurality of segmented regions may extend radially outward from a center of the lens portion, and have different radii of curvature and thus have different refractive powers. The refractive power of the segmented region through which the on-axis rear cross-section passes may be greater than the refractive power of the segmented region through which the on-axis front cross-section passes.

As described above, the intraocular lens having a plurality of segmented regions makes it easier to obtain an appropriate visual field even when the wearer's pupil becomes smaller. Furthermore, by designing the intraocular lens to have the refractive power of the segmented region through which the on-axis rear cross-section passes is greater than the refractive power of the segmented region through which the on-axis front cross-section passes, an increase in volume of the vicinity of the proximal end portion of the rear supporter that is close to the pushing member is further appropriately suppressed.

A virtual partial cross-section that is perpendicular to the lens surface of the lens portion and extends in an arbitrary direction outward from the geometric center of the lens portion is assumed. In this case, the cross-sectional area of the on-axis rear cross-section may be less than or equal to the cross-sectional area of a virtual partial cross-section in another direction. In this case, an increase in volume near the proximal end portion of the rear supporter adjacent to the pushing member can be further suppressed. Therefore, when the intraocular lens is pushed out in the passage of the nozzle by the pushing member, the intraocular lens can be more appropriately folded.

It should be noted that the intraocular lens may be designed such that the refractive power of the lens portion in the on-axis rear cross-section is greater than the refractive power of the lens portion in a virtual cross-section in another direction. In other words, the intraocular lens may be designed such that the radius of curvature of the lens portion in the on-axis rear cross-section is smaller than the radius of curvature of the lens portion in the virtual cross-section in the other direction. In this case, the intraocular lens is easily folded into a small size near the proximal end of the rear supporter where the pushing member is located.

When a plurality of segmented regions are formed on at least one of the front surface and the rear surface of the lens portion, the on-axis rear cross-section may pass through a segmented region having the largest refractive power among the plurality of segmented regions. In this case, an increase in volume near the proximal end portion of the rear supporter adjacent to the pushing member can be further suppressed.

Note that the present disclosure shows an intraocular lens that includes one or more supporters (a pair of supporters in the present disclosure) that extend outward from the lens portion to have a curved shape. As described above, by making the cross-sectional area of the lens portion in the second cross-section in the direction passing through the proximal end portion of each of the pair of supporters greater than or equal to the cross-sectional area in a virtual cross-section in another direction, rigidity at the proximal ends of the supporters can be secured. Further, by making the cross-sectional area of the on-axis rear section extending rearward from the geometric center along the pushing axis smaller than the cross-sectional area of the on-axis front cross-section extending forward from the geometric center along the pushing axis, an increase in volume of the proximal end portion of the rear supporter can be also prohibited. However, some of the techniques exemplified in this disclosure may be applied to an intraocular lens without a supporter that curves outwardly from the lens portion (e.g., an intraocular lens with a plate-shaped supporter). For example, even in a plate-type intraocular lens, by making the cross-sectional area of the lens portion in a virtual cross-section (a first cross-section) in a direction perpendicular to the pushing axis equal to or less than the cross-sectional area in a virtual cross-section in another direction, the maximum value of the cross-sectional area of the lens portion in a direction perpendicular to the pushing axis can be reduced.

### <Second aspect>

A second aspect of an intraocular lens according to the present disclosure will be described. The intraocular lens exemplified in the present disclosure includes a disc-shaped lens portion and one or more supporters that curve outwardly from the lens portion and are deformable. The intraocular lens exemplified in the present disclosure is inserted into an eye of a patient from an insertion port at a front end of the intraocular lens insertion instrument through an internal passage of an intraocular lens insertion instrument. The intraocular lens insertion instrument includes a pushing member and a nozzle. By moving forward in the passage along a pushing axis, which is an axis of the passage of the intraocular lens insertion, the pushing member may contact a rear supporter that is the supporter extending rearward from the lens portion and may push out the intraocular lens forward while folding the rear supporter onto the lens portion. The nozzle has the passage that is tapered toward the front side and includes the insertion port at its front end. After the intraocular lens was folded while the pushing member pushed the intraocular lens forward through the passage, the intraocular lens is discharged through the insertion port. A virtual partial cross-section that is perpendicular to the lens surface of the lens portion and extends in an arbitrary direction outward from the geometric center of the lens portion is assumed. In the intraocular lens in the present disclosure, the cross-sectional area of the lens portion in the virtual partial cross-section changes depending on the direction in which the virtual partial cross-section is defined. When the intraocular lens is folded by the nozzle, the cross-sectional area of the on-axis rear cross-section, which is a virtual partial cross-section extending rearward from the geometric center along the pushing axis, becomes less than or equal to the cross-sectional area of a virtual partial cross-section in another direction.

As described above, if the intraocular lens can be folded into a small size within the nozzle passage, the load generated when pushing out the intraocular lens in the nozzle passage would be reduced, and it would also be easier to reduce the nozzle diameter. The inventors of the present disclosure had thought that the intraocular lens could be folded into a small size in the nozzle by designing the intraocular lens such that a cross-section of the intraocular lens that passes through the geometric center of the lens portion and is perpendicular to the pushing axis (hereinafter, referred to as a "vertical cross-section") has a small cross-sectional area. However, after repeated trials, the inventor found that even if the cross-sectional area of the intraocular lens in the above-mentioned vertical cross-section was made small, if the intraocular lens with a rear supporter being tacked was pushed out in the nozzle by the pushing member, load increased when the vicinity of the proximal end portion of the rear support passed through the nozzle. This is because the volume of the pushing member that is pushing out the intraocular tens while being in contact with the the rear supporter is added to the increased volume of the vicinity of the proximal end portion due to the bent of the proximal end portion of the rear supporter. As a result, it would be difficult to bend the vicinity of the proximal end portion of the rear supporter into a small size.

On the contrary, in the intraocular lens of the present disclosure, the cross-sectional area of the on-axis rear cross-section extending rearward from the geometric center along the pushing axis of the lens portion is less than or equal to the cross-sectional area of a virtual partial cross-section in another direction. As a result, an increase in volume of the vicinity of the proximal end portion of the rear supporter that is close to the pushing member is prevented, and therefore the vicinity of the proximal end portion of the rear supporter is easily bent to a small size. Therefore, the intraocular lens in the present disclosure is more easily folded into a smaller size when being pushed out by the pushing member along the passage of the nozzle of the intraocular lens insertion instrument.

In the present disclosure, the intraocular lens having two loop-shaped (curved) supporters each having a free end is exemplified. However, even in an intraocular lens having three or more loop-shaped supporters, an increase in volume of the vicinity of the proximal end portion of the rear supporter can be prevented by making the cross-sectional area of the on-axis rear cross-section smaller than the cross-sectional area of the on-axis front cross-section,

It should be noted that the intraocular lens may be designed such that the refractive power of the lens portion in the on-axis rear cross-section is equal to or greater than the refractive power of the lens portion in a virtual cross-section in another direction. In the lens portion, the radius of curvature of the portion with high refractive power is smaller than the radius of curvature of the portion with low refractive power. In other words, in the lens portion, the curve of the lens surface in the portion where the refractive power is large is steeper than the curve of the lens surface in the portion where the refractive power is small. Here, since it is optically undesirable to form a step at the geometric center of the lens portion, the lens portion at the geometric center through which each virtual partial cross-section passes has the same thickness regardless of the direction of the assumed virtual partial cross-section. As a result, the thickness of the side surface (an edge portion) of the lens portion in an area where the refractive power is large (i.e., an area where the curve of the lens surface is steep) is smaller than that of the lens portion where the refractive power is small (i.e., an area where the curve of the lens surface is gentle). Therefore, the greater the refractive power in the virtual partial cross-section extending outward from the geometric center is, the smaller the cross-sectional area of the lens portion in the virtual partial cross-section. Therefore, by designing the intraocular lens such that the refractive power of the lens portion in the on-axis rear cross-section is greater than or equal to the refractive power of the lens portion in a virtual partial cross-section in another direction, the cross-sectional area of the lens portion in the on-axis rear cross-section is appropriately reduced. As a result, an increase in volume of the vicinity of the proximal end portion of the rear supporter that is close to the pushing member is prevented, and therefore the vicinity of the proximal end portion of the rear supporter is easily bent to a small size.

Note that it is also possible to change the configuration of the lens portion in the on-axis rear cross-section. For example, the intraocular lens may be designed such that the radius of curvature of the lens portion in the on-axis rear cross-section is equal to or smaller than the radius of curvature of the lens portion in the virtual cross-section in the other direction. In this case, an increase in volume near the proximal end portion of the rear supporter adjacent to the pushing member can be suppressed.

Further, when the intraocular lens is a toric intraocular lens that corrects astigmatism of a wearer, a strong principal meridian and a weak principal meridian exist in the lens portion. The strong principal meridian refers to a meridian among arbitrary meridians that has a small radius of curvature and has the maximum refractive power. In addition, the weak principal meridian refers to a meridian with a large radius of curvature and the minimum refractive power. The toric intraocular lens may be designed such that the position of the on-axis rear cross-section is aligned with the strong principal meridian of the lens portion. That is, in a toric intraocular lens, the strong principal meridian of the lens portion may be aligned with the pushing axis of the intraocular lens. In this case, an increase in volume near the proximal end portion of the rear supporter adjacent to the pushing member can be suppressed.

The intraocular lens may be designed such that at least one of a pair of virtual partial cross-sections extending in a direction perpendicularly to the pushing axis from the geometric center (hereinafter, referred to as a "pair of vertical partial cross-sections") has a cross-sectional area equal to or less than a cross-sectional area of a virtual partial cross-section in another direction. In other words, the cross-sectional area of at least one of the pair of vertical partial cross-sections may match the cross-sectional area of the on-axis rear cross-section. Considering load generated when the entire intraocular lens passes through the nozzle passage, it is also preferable to minimize the maximum value of the cross-sectional area of the lens portion in a direction perpendicular to the pushing axis. By setting the cross-sectional area of at least one of the pair of vertical partial cross-sections to be less than or equal to the cross-sectional area of the virtual partial cross-section in the other direction, the maximum value of the cross-sectional area of the lens portion in the direction perpendicular to the pushing axis is also reduced. As a result, in addition to the load generated when the vicinity of the proximal end portion of the rear supporter passes through the nozzle, the load generated when the geometric center of the lens portion passes through the nozzle is also appropriately reduced. Therefore, when the intraocular lens is pushed out in the passage of the nozzle by the pushing member, the intraocular lens of the present disclosure can be folded into a smaller size.

As described above, the greater the refractive power in the virtual partial cross-section extending outward from the geometric center is, the smaller the cross-sectional area of the lens portion in the virtual partial cross-section. Therefore, the intraocular lens may be designed such that the refractive power of the lens portion in at least one of the pair of vertical partial cross-sections is greater than or equal to the refractive power of the lens portion in a virtual partial cross-section in another direction. In this case, the maximum value of the cross-sectional area of the lens portion in the direction perpendicular to the pushing axis is appropriately reduced.

Furthermore, the smaller the radius of curvature in the virtual partial cross-section extending outward from the geometric center is, the smaller the cross-sectional area of the lens portion in the virtual partial cross-section. Therefore, the intraocular lens may be designed such that the radius of curvature of the lens portion in at least one of the pair of vertical partial cross-sections is greater than or equal to the radius of curvature of the lens portion in a virtual partial cross-section in another direction. In this case, the maximum value of the cross-sectional area of the lens portion in the direction perpendicular to the pushing axis is appropriately reduced.

A plurality of segmented regions may be formed on at least one of the front surface and the rear surface of the lens portion. The plurality of segmented regions may extend radially outward from a center of the lens portion, and have different radii of curvature and thus have different refractive powers. The on-axis rear cross-section may pass through a segmented region having the largest refractive power among the plurality of segmented regions.

In the intraocular lens having a plurality of segmented regions, unlike an intraocular lens in which a plurality of regions are concentrically arranged, even if the wearer's pupil becomes smaller, light passes through each of the plurality of segmented regions and is focused on the retina. Therefore, the advantageous effects of the multifocal intraocular lens can be appropriately obtained regardless of the size of the wearer's pupil. Furthermore, the plurality of segmented regions have different radii of curvature and thus have different refractive powers. Therefore, the amount of light scattered in unintended directions is reduced as compared to a situation where a group of micro prisms or the like is formed in the segmented region. As a result, loss of light reaching the retina is less likely to occur, making it easier to obtain a better visual field. In addition, it is easy to process the intraocular lens with high precision and is less likely to be damaged when inserted into an eye.

Furthermore, as described above, in each part of the lens portion of the intraocular lens, the greater the refractive power (the smaller the radius of curvature) is, the smaller the cross-sectional area of the lens portion is. Therefore, by designing the intraocular lens such that the on-axis rear cross-section passes through the segmented region with the largest refractive power among the plurality of segmented regions, the volume of the vicinity of the proximal end portion of the rear supporter adjacent to the pushing member is reduced. increase is suppressed. Therefore, the vicinity of the proximal end portion of the rear supporter is easily bent into a small size.

The intraocular lens may be designed such that at least one of the pair of vertical partial sections passes through a segmented region with the largest refractive power among the plurality of segmented regions. In this case, the maximum value of the cross-sectional area of the lens portion in the direction perpendicular to the pushing axis is also reduced. As a result, in addition to the load generated when the vicinity of the proximal end portion of the rear supporter passes through the nozzle, the load generated when the geometric center of the lens portion passes through the nozzle is also appropriately reduced.

However, as described above, the technique illustrated in the present disclosure may also be applied to an intraocular lens in which a plurality of segmented regions are not formed (for example, a toric intraocular lens). Further, both the plurality of segmented regions and a toric surface for correcting astigmatism in the wearer may be formed in the lens portion. Note that among the front and rear surfaces of the lens surface, the surface on which the segmented region is formed and the surface on which the toric surface is formed may be the same surface or may be different surfaces.

### BRIEF DESCRIPTION OF DRAWINGS

FIG. 1 is a plan view of an intraocular lens.
FIG. 2 is a perspective view of an intraocular lens insertion instrument viewed diagonally from a right-upper side.
FIG. 3 is a perspective view of a plunger viewed diagonally from a right-upper side.
FIG. 4 is a schematic explanatory diagram showing a state in which the intraocular lens has been appropriately moved and deformed.
FIG. 5 is a plan view showing one example of the intraocular lens according to a first embodiment in which a plurality of segmented regions are formed.
FIG. 6 is a diagram schematically comparing cross-sectional views of the distant vision region and the near vision region, which are taken through the geometric center of the lens portion and perpendicular to the lens surface of the lens portion.
FIG. 7 is a diagram comparing MTF curves of four intraocular lenses with different refractive powers and central angles in a distant vision region, a near vision region, and an intermediate vision region.
FIG. 8 is a diagram comparing MTF curves of the intraocular lens when no transition portion is formed between the distant vision region and the near vision region and when the transition region is formed between the distant vision region and the near vision region.
FIG. 9 is a diagram showing lines for calculating a total value of refractive powers, an on-axis rear cross-section, and a position of an on-axis front cross-section in the intraocular lens shown in FIG. 5.
FIG. 10 is a plan view showing one example of a modification of the intraocular lens shown in FIG. 5.
FIG. 11 is a plan view showing one example of the intraocular lens according to a second embodiment in which a plurality of segmented regions are formed.
FIG. 12 is a diagram showing positions of a specific virtual cross-section and a specific virtual partial cross-section in the intraocular lens shown in FIG. 11.

### DETAILED DESCRIPTION

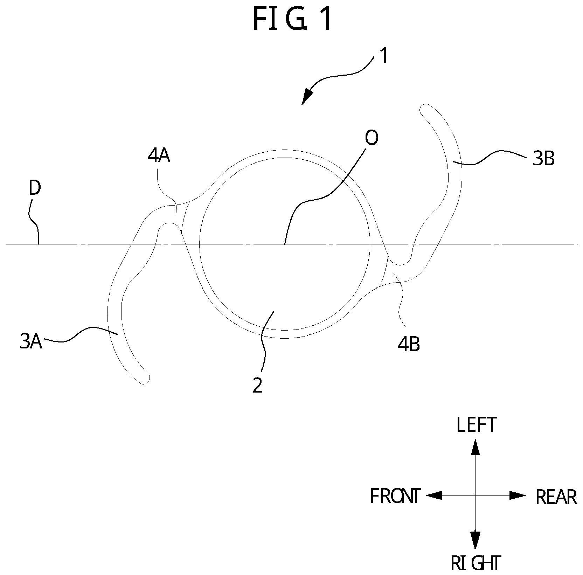

Hereinafter, a plurality of typical embodiments according to the present disclosure will be described with reference to the drawings. An intraocular lens 1 in this embodiment is inserted into a patient's eye using an intraocular lens insertion instrument 100. In the following description, the direction of the intraocular lens 1 that is inserted into the eye first by the intraocular lens insertion instrument 100 (i.e., the left side in FIG. 1) is defined as a front side of the intraocular lens 1.

### (Schematic configuration of the intraocular lens)

With reference to FIG. 1, a schematic configuration of the intraocular lens 1 in this embodiment will be described. The intraocular lens 1 includes a lens portion 2 and supporters 3. The intraocular lens 1 in this embodiment is a so-called one-piece type intraocular lens in which the lens portion 2 and the supporters 3 are integrally formed. However, at least one of techniques exemplified in the present disclosure may also be applied to a so-called three-piece intraocular lens in which the lens portion 2 and the supporters 3 are separately formed from each other. The intraocular lens 1 is deformable. The intraocular lens 1 may be made of various soft materials, such as simple materials such as BA (butyl acrylate) and HEMA (hydroxyethyl methacrylate), and composite materials of acrylic ester and methacrylic ester.

The lens portion 2 provides a predetermined refractive power to the eye of a wearer who wears the intraocular lens 1 (that is, the patient's eye). Details of the refractive power of the lens portion 2 will be described later. The shape of the lens portion 2 is a disk shape. The optical axis of the lens portion 2 exemplified in this embodiment passes through the geometric center O of the lens portion 2 and extends in a direction perpendicular to the lens surface of the lens portion 2 (i.e., extends in a vertical direction). However, the optical axis of the lens portion 2 does not necessarily need to be aligned with the geometric center O of the lens section.

The intraocular lens 1 in this embodiment includes a pair of supporters 3 (front supporter 3A and a rear supporter 3B). The proximal end portions 4 of the pair of supporters 3 (that is, the proximal end portion 4A of the front supporter 3A and the proximal end portion 4B of the rear supporter 3B) are connected to different parts of the side surface of the outer peripheral portion of the lens portion 2 (in this embodiment, connected to opposite portions of the outer circumferential portion of the lens portion 2 which has a disk shape). When the intraocular lens 1 is worn within the patient's eye, the pair of supporters 3 support the lens portion 2 within the patient's eye.

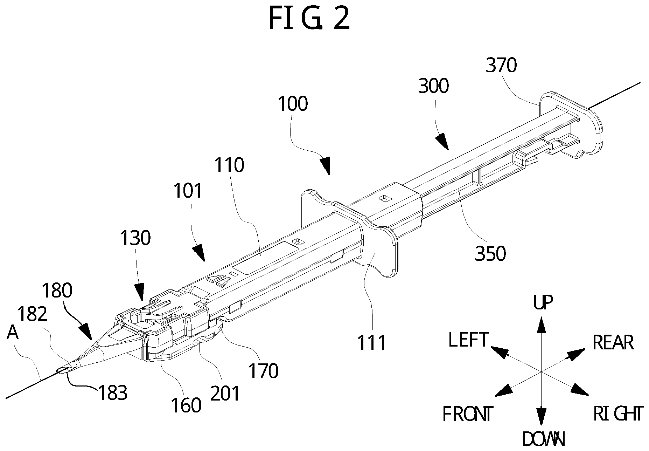

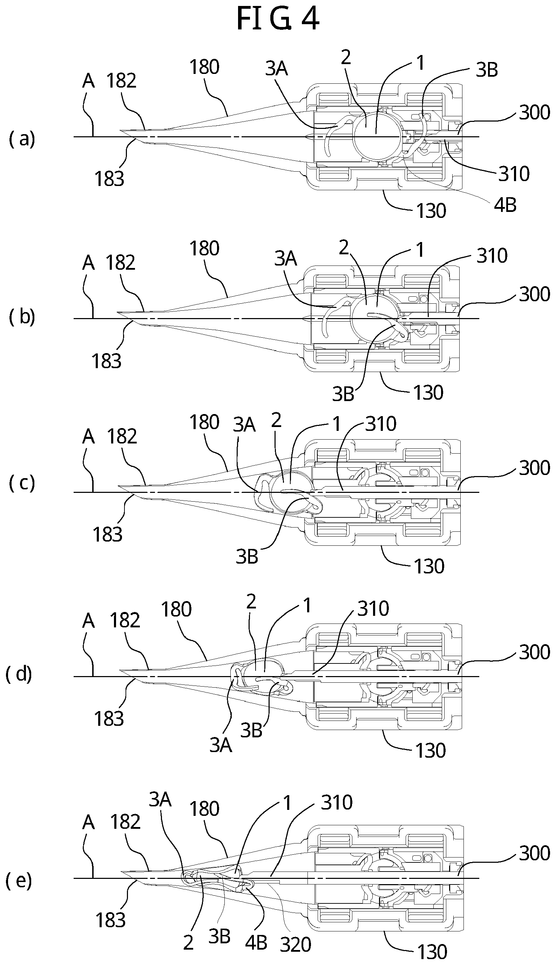

Although the details will be described later, in this embodiment, the direction of movement D of the intraocular lens 1 in which the intraocular lens 1 moves through the passage of a nozzle 180 (see FIGS. 2 and 4) of the intraocular lens insertion instrument 100 is set in advance. In other words, when the intraocular lens 1 advances in the passage of the nozzle 180, the direction of movement D of the intraocular lens 1 matches an pushing axis A(see FIGS. 2 and 4) which is an axis of the passage of the nozzle 180.

The front supporter 3A is curved toward the front of the intraocular lens insertion instrument 100 from the side surface of the outer peripheral portion of the lens portion 2 when the intraocular lens 1 is installed in an installation portion 130 of the intraocular lens insertion instrument 100 (see FIG. 4). That is, the front supporter 3A has a loop shape curved in a circumferential direction, and the front end portion of the front supporter 3A is a free end. The rear supporter 3B is curved toward the rear side of the intraocular lens insertion instrument 100 from the outer peripheral portion of the lens portion 2 when the intraocular lens 1 is installed in the installation portion 130 of the intraocular lens insertion instrument 100 (see FIG. 4). That is, the rear supporter 3B has a loop shape curved in a circumferential direction, and the tip end of the rear supporter 3B is also a free end.

However, at least some of the techniques exemplified in the present disclosure may also be applied to an intraocular lens with a supporter having a shape different from the loop shape (for example, an intraocular lens with a plate-shaped supporter, etc.). Furthermore, at least a portion of the techniques exemplified in the present disclosure may be applied to an intraocular lens including three or more loop-shaped supporters.

### (Intraocular lens insertion instrument)

An intraocular lens insertion instrument 100 will be described. In the following description, the direction of the nozzle 180 side of a main body 101 of the intraocular lens insertion instrument 100 (i.e., a lower left side in FIG. 2) is defined as a front side for the intraocular lens insertion instrument 100, and the direction of a pressing portion 370 of a plunger 300 (i.e., a upper right side in FIG. 2) is defined as a rear side for the intraocular lens insertion instrument 100. Furthermore, the upper side in FIG. 2 is defined as an upper side for the intraocular lens insertion instrument 100, the lower side in FIG. 2 is defined as a lower side for the intraocular lens insertion instrument 100, the lower right side in FIG. 2 is defined as a right side for the intraocular lens insertion instrument 100, and the upper left side in FIG. 2 is defined as a left side for the intraocular lens insertion instrument 100.

First, with reference to FIG. 2, an overall configuration of the intraocular lens insertion instrument 100 in this embodiment will be described. As mentioned above, the intraocular lens insertion instrument 100 is used to insert the deformable intraocular lens 1 into an eye. The intraocular lens insertion instrument 100 includes the main body 101 and the plunger 300. The main body 101 has a substantially cylindrical shape, and the intraocular lens 1 is inserted into the eye through a passage inside the main body 101. The plunger 300 has a rod shape and is movable in the front and rear directions inside the passage in the main body 101. The plunger 300 pushes out the intraocular lens 1 filled inside the main body 101 by moving forward along the pushing axis (i.e., an axis of the passage) A.

The main body 101 and plunger 300 in this embodiment are made of resin material. The intraocular lens insertion instrument 100 may be formed by molding, cutting by carving out resin, or the like. Since the intraocular lens insertion instrument 100 is made of resin material, a user can easily discard the used intraocular lens insertion instrument 100.

In this embodiment, a lubricant coating treatment is applied to an inner wall of the main body 101 in order to smoothly insert the soft intraocular lens 1 having adhesive properties into the eye. Moreover, the intraocular lens insertion instrument 100 in this embodiment is formed to be colorless and transparent or colorless and semitransparent. Therefore, the user can easily visually check the state of the intraocular lens 1 filled inside the intraocular lens insertion instrument 100 from outside the intraocular lens insertion instrument 100.

The main body 101 will be described with reference to FIG. 2. The main body 101 includes a main body cylinder 110, the installation part 130, and the nozzle (i.e., an insertion portion) 180.

The main body cylinder 110 is formed in a cylindrical shape extending in a front-rear direction, and is located on the rear end side (i.e., a base end side) of the main body 101. A projecting portion 111 that is grasped by a user is formed on the outer circumference of the main body cylinder 110 at a position slightly forward of the rear end of the main body cylinder 110.

The installation portion 130 is connected to the front end side of the main body cylinder 110. The intraocular lens 1 is installed (filled) in the installation portion 130. Specifically, the installation portion 130 includes a holding portion 160 and a setting portion 170. The holding portion 160 holds the intraocular lens 1 when the intraocular lens insertion instrument 100 is not used. The setting portion 170 is rotatable about the axis of the tip end of the setting portion 170. When the setting portion 170 is rotated, the intraocular lens 1 held by the holding portion 160 is moved to a standby position where the intraocular lens 1 is to be pushed out by the plunger 300. The intraocular lens 1 is positioned (i.e., fixed) at the standby position.

The nozzle 180 is connected to the front end side of the installation portion 130. The passage area within the nozzle 180 decreases toward the front side because the intraocular lens 1 is deformed to get smaller in the process of pushing the intraocular lens 1 forward. That is, the nozzle 180 has a tapered internal space therein. A cylindrical insertion portion 182 with an obliquely cut tip is disposed at the front end of the nozzle 180. The insertion portion 182 is inserted into the eye. An insertion port 183 is formed at the front end of the insertion portion 182. The insertion port 183 is an opening for discharging the intraocular lens 1 forward from the internal passage. The passage inside the main body 101 extends from the rear end of the main body cylinder 110 to the insertion opening 183 at the front end of the nozzle 180.

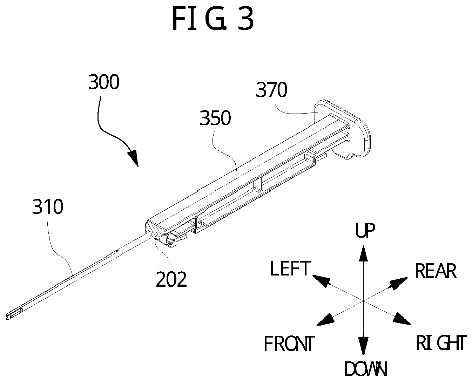

A schematic configuration of the plunger 300 will be described with reference to FIG. 3. The plunger 300 in this embodiment includes a pushing member 310, a shaft base 350, and a pressing portion 370.

The pressing portion 370 is formed at a rear end of the plunger 300. The pressing portion 370 is a plate-shaped member extending in a direction perpendicular to the pushing axis A (see FIG. 2). The user's finger comes into contact with the pressing portion 370 when the user pushes the plunger 300 forward.

The shaft base 350 is a rod-shaped member that extends forward from the front end side of the pressing portion 370. In this embodiment, the shaft base 350 is formed so that the shaft base 350 has an approximately H-shaped cross-section that extends perpendicular to the pushing axis A. By inserting the shaft base 350 into the main body cylinder 110 whose cross-section perpendicular to the pushing axis A is approximately rectangular, circumferential rotation of the pushing axis A of the plunger 300 with respect to the main body 101 is prohibited. When the plunger 300 moves forward and reaches the position at which insertion of the intraocular lens 1 into the eye is completed, a tilted surface at the lower front end of the shaft base 350 comes into contact with a tilted surface of the main body 101. As a result, the front end of plunger 300 is prevented from protruding excessively from insertion port 183 (see FIG. 2).

The pushing member 310 is a rod-shaped member, and extends forward from the front end of the shaft base 350 along the pushing axis A. The pushing member 310 is formed so that the cross-section of the pushing member 310 perpendicular to the pushing axis A has a substantially circular shape. Further, the pushing member 310 has a thickness that allows the pushing member 310 to pass through the insertion opening 183 of the main body 101. The pushing member 310 moves forward in the passage of the main body 101 along the pushing axis A, thereby pushing the intraocular lens 1 forward while folding the lens 1 into a small size and discharging the lens 1 into the eye through the insertion port 183.

With reference to FIG. 4, movement and deformation of the intraocular lens 1 when the intraocular lens 1 is inserted into the eye using the intraocular lens insertion instrument 100 in this embodiment will be described. First, an operator rotates the setting portion 170 (see FIG. 2) to the standby position where the intraocular lens 1 held by the holding portion 160 (see FIG. 2) of the installation portion 130 can be pushed out by the plunger 300. Here, as shown in FIG. 4(a), the proximal end 4B of the rear supporter 3B of the intraocular lens 1 held by the holding portion 160 is displaced in either the left or right direction with respect to the pushing axis A.

Next, the operator uses a syringe or the like to inject a lubricant (viscoelastic substance) into the installation portion 130 and starts moving the plunger 300 forward. As a result, the pushing member 310 comes into contact with the rear supporter 3B of the intraocular lens 1, as shown in FIG. 4(a).

When the plunger 300 is further pushed forward, the rear supporter 3B is moved (bended) in a direction approaching the lens portion 2 by the pushing member 310, as shown in FIG. 4(b). As a result, the rear supporter 3B is deformed and moved above the lens portion 2, and the tip of the rear supporter 3B faces forward. The state shown in FIG. 4(b) may also be referred to as a state in which the rear supporter 3B is tacked. In addition, in this embodiment, the rear supporter 3B is tacked when the pushing member 310 is pushed forward. However, the technology of the present disclosure may also be applied when the entire intraocular lens 1 is moved forward by the pushing member 310 after tucking of the rear supporter 3B is performed by a member different from the pushing member 310.

When the plunger 300 is pushed further forward, the pushing member 310 comes into contact with the lens portion 2, and the entire intraocular lens 1 moves forward. As shown in FIG. 4(c), when the intraocular lens 1 reaches the nozzle 180, the front supporter 3A comes into contact with the tapered inner wall of the nozzle 180. As a result, the front supporter 3A is deformed and moved above the lens portion 2, and the tip of the front supporter 3A faces rearward. That is, tucking of the front supporter 3A is performed. Further, by the front supporter 3A coming into contact with the tapered inner wall of the nozzle 180, the direction of movement D of the intraocular lens 1, which is assumed in advance (see FIG. 1), is substantially aligned with the pushing axis A of the intraocular lens insertion instrument 100.

When the plunger 300 is pushed further forward, the lens portion 2 of the intraocular lens 1 also comes into contact with the tapered inner wall of the nozzle 180. As a result, as shown in FIG. 4(d), the lens portion 2 of the intraocular lens 1 is folded into a smaller size.

As shown in FIG. 4(e), the size of the intraocular lens 1 decreases as the lens 1 is pushed to move forward. Since the passage area of the nozzle 180 narrows toward the front side, the load (or a pressure) on the intraocular lens 1 gradually increases. For example, a large load is likely to be applied to the portion where the cross-sectional area of the lens portion 2 in the direction perpendicular to the pushing axis A has a maximum value (the portion near the geometric center O (see FIG. 1) of the lens portion 2). Furthermore, in the vicinity of the proximal end 4B of the rear supporter 3B, the volume of the pushing member 310 is further added to the volume increased by bending the proximal end 4B of the rear supporter 3B. Therefore, a large load is likely to be applied to the vicinity of the proximal end 4B of the rear supporter 3B. However, the intraocular lens 1 in this embodiment is designed to be easily folded into a smaller size. The detail of this mechanism will be described later.

### (Segmented region of the lens portion)

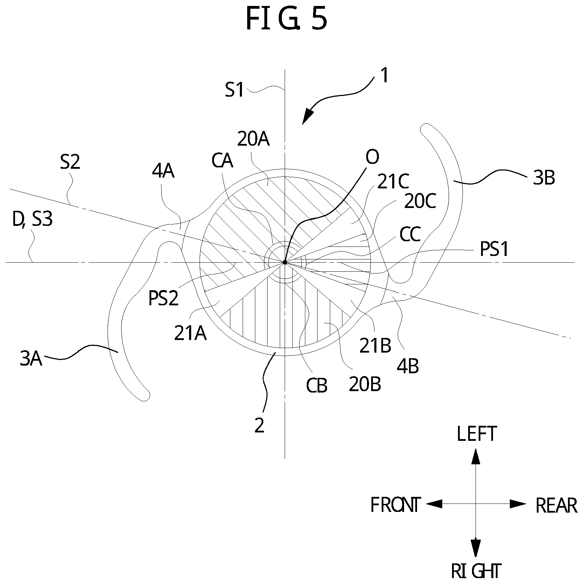

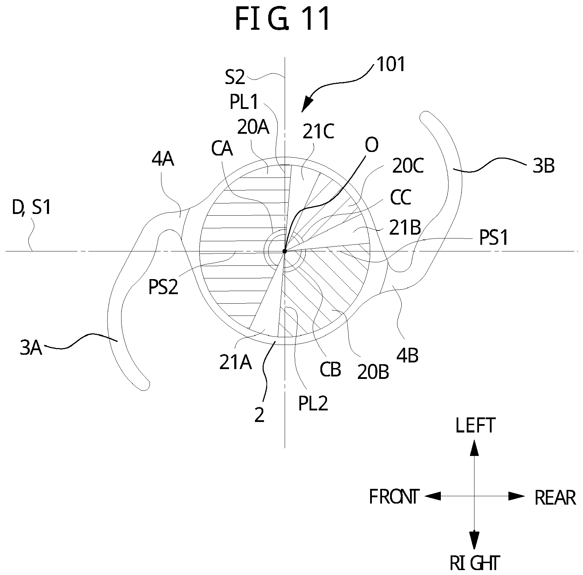

A segmented region 20 formed in the lens portion 2 of the intraocular lens 1 in this embodiment (a first embodiment and a second embodiment) will be described. As shown in FIGS. 5 and 11, in the intraocular lens 1 in this embodiment, three or more segmented regions 20 (20A, 20B, 20C) are provided on at least one of the front and rear surfaces of the disc-shaped lens portion 2.

The plurality of segmented regions 20 have different refractive powers. Specifically, the lens portion 2 in this embodiment has a distant vision region 20A, a near vision region 20B, and an intermediate vision region 20C. The distant vision region 20A has the smallest refractive power among the plurality of segmented regions 20. The near vision region 20B has the largest refractive power among the plurality of segmented regions 20. The intermediate vision region 20C has a refractive power between the refractive power of the distant vision region 20A and the refractive power of the near vision region 20B. Therefore, the light that has passed through the distant vision region 20A, the near vision region 20B, and the intermediate vision region 20C in the lens portion 2 is focused on the wearer's retina, thereby providing a multifocal effect. Note that in the present disclosure, each segmented region 20 is a region in which the refractive power has substantially the same value regardless of the location within the region.

When the lens portion 2 is viewed in a direction perpendicular to the lens surface, the plurality of segmented regions 20 spread radially outward from the center of the lens portion 2. That is, each of the segmented regions 20 is segmented by boundary lines each extending linearly outward from one point in the center portion of the lens portion 2. Therefore, unlike an intraocular lens in which a plurality of regions are arranged concentrically, light easily passes through all the plurality of segmented regions 20 even when the wearer's pupil becomes smaller. As a result, the multifocal effect can be easily obtained regardless of the size of the wearer's pupils. Note that the boundary lines of the segmented regions 20 are not limited to straight lines, but may be curved lines or the like, or may be bent.

In detail, in the intraocular lens 1 in this embodiment, the lens 1 spreads radially outward from one reference point in the center portion of the lens portion 2 (in this embodiment, the geometric center O of the lens portion 2). In other words, in each segmented region 20, all straight boundary lines (ends) extending outward from the center of the lens portion 2 pass through the same reference point. Therefore, even if the wearer's pupil becomes smaller, the amount of light passing through each of the plurality of segmented regions 20 can be easily secured as compared with a situation where a fixed region (for example, a circular region) is separately formed at the center of the lens portion 2. As a result, a multifocal effect can be easier obtained regardless of the size of the wearer's pupil, so the wearer's field of vision becomes easier to improve. However, a certain area at the center of the lens portion 2 may be formed. Even in this case, it is easier to obtain the multifocal effect regardless of the size of the wearer's pupils as compared to a situation where a plurality of regions are arranged concentrically.

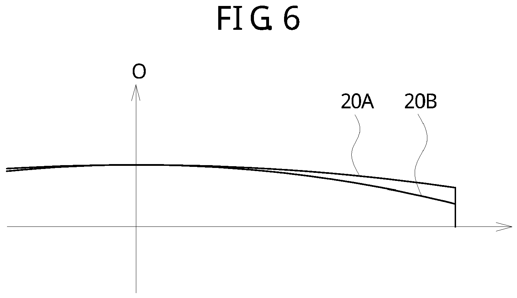

FIG. 6 is a diagram schematically comparing cross-sectional views of the distant vision region and the near vision region, which are taken through the geometric center of the lens portion and perpendicular to the lens surface of the lens portion. As shown in FIG. 6, the plurality of segmented regions 20 formed in the intraocular lens 1 in this embodiment have different radii of curvature and thus have different refractive powers. Note that the lens surface of each segmented region 20 may be a spherical surface or an aspherical surface. When the lens surface is an aspherical surface, the term "radius of curvature" in the present disclosure may be interpreted as the radius of curvature of a spherical surface approximated to the aspherical lens surface.

In the example shown in FIG. 6, the radius of curvature of the distant vision region 20A with low refractive power is larger than the radius of curvature of the near vision region 20B with high refractive power. In other words, the curve of the lens surface in the distant vision region 20A, which has a small refractive power, is gentler than the curve of the lens surface in the near vision region 20B, which has a large refractive power. By changing the radius of curvature of each of the plurality of segmented regions 20 to change the refractive power of each segmented region 20, compared to the situation where different microprism groups or the like are formed in each of the segmented regions 20 to change the refractive power, the amount of light scattered in unintended directions is reduced. As a result, loss of light reaching the retina is less likely to occur, making it easier to obtain a better visual field. In addition, the intraocular lens 1 in this embodiment is easily processed with high accuracy, and there is a low possibility that the lens 1 is damaged when inserted into the eye.

Note that, as shown in FIG. 6, no step or the like is provided at the geometric center O of the lens portion 2. Therefore, the thickness of the side surface (an edge part) of the lens portion 2 in a part where the refractive power is large (i.e., the part where the curve of the lens surface is gentle) is thinner than the thickness of the side surface (an edge part) of the lens portion 2 in a part where the refractive power is small (i.e., the part where the curve of the lens surface is gentle).

As shown in FIGS. 5 and 11, when the lens portion 2 is viewed in a direction perpendicular to the lens surface, transition portions 21 (21A, 21B, 21C) between the plurality of segmented regions 20 in the lens portion 2 are formed. Each transition portion 21 has a radius of curvature that continuously changes from the end of one of a pair of segmented regions 20 adjacent to each other via the transition portion 21 to the end of the other one of the pair of segmented regions 20. As a result, the ends of the pair of segmented regions 20 are smoothly connected to each other.

In the example shown in FIGS. 5 and 11, the refractive power of the distant vision region 20A is 0D, the refractive power of the near vision region 20B is +3.25D, and the refractive power of the intermediate vision region 20C is +2.0D. Therefore, in the transition portion 21Aformed between the distant vision region 20A and the near vision region 20B, the refractive power changes from 0D to +3.25D. In the transition portion 21B formed between the near vision region 20B and the intermediate vision region 20C, the refractive power smoothly changes from +3.25D to +2.0D from the end close to the near vision region 20B to the end close to the intermediate vision region 20C. In the transition portion 21C formed between the intermediate vision region 20C and the distant vision region 20A, the refractive power smoothly changes from +2.0D to 0D from the end of the intermediate vision region 20C to the end of the distant vision region 20A.

If the transition portion 21 is not formed in the lens portion 2, a step or the like may be formed at the boundary between a pair of adjacent segmented regions 20, and such a step would cause a phenomenon in which light is diffusely reflected by the step or the like, deteriorating the visual field (for example, a halo or glare, etc.). On the contrary, by forming the transition portion 21 between a pair of adjacent segmented regions 20, the influence of diffused reflection of light due to a step or the like is less likely to occur. Furthermore, when the transition portion 21 is formed in the lens portion 2, the refractive power of each region within the transition portion 21 smoothly shifts from the refractive power of one of the adjacent segmented regions 20 to the refractive power of the other of the segmented regions 20. As a result, in addition to the refractive power of each segmented region 20, a refractive power between the refractive powers of the segmented regions 20 is also applied to the lens portion 2. Therefore, by forming the transition portions 21 in the lens portion 2, the effect of expanded depth of focus (EDOF) of the multifocal intraocular lens can also be appropriately obtained.

In the intraocular lens 1 according to the present embodiment, both the segmented region 20 and the transition portion 21 refract light, in a direction toward the optical axis, that is incident on the lens portion 2 in parallel with the optical axis of the lens portion 2. As a result, the amount of light that is lost without reaching the retina after passing through the lens portion 2 is reduced. Therefore, it becomes easier to obtain a better field of view.

The central angle of each of the transition portions 21 extending outward from the central portion of the lens portion 2 (in this embodiment, the geometric center O, which is a reference point) is set to 5 degrees or more and 30 degrees or less. In this case, both the multifocal effect by the plurality of segmented regions 20 and the depth of focus expansion effect can be appropriately obtained. In addition, it is more preferable that the central angle of each of the transition portions 21 is set to 15 degrees or more and 20 degrees or less. As one example, the central angles of the three transition portions 21 formed in the intraocular lens 1 illustrated in FIG. 5 are all set to 20 degrees. However, when forming a plurality of transition portions 21 in the lens portion 2, the central angle of each of the transition portions 21 does not necessarily need to be the same with each other.

For the distant vision region 20A, the near vision region 20B, and the intermediate vision region 20C, which spread outward from the center of the lens portion 2, the central angle CA of the distant vision region 20A is largest, and the central angle CAof the intermediate vision region 20C is smallest. In this case, distant vision using the distant vision region 20A and near vision using the near vision region 20B can be appropriately obtained with expanded depth of focus.

Specifically, the central angle CA of the distant vision region 20A is set to be 140 degrees or more, the central angle CB of the near vision region 20B is set to be 90 degrees or more, and the central angle CC of the intermediate vision region 20C is set to be 30 degrees or more. In this case, distant vision using the distant vision region 20A and near vision using the near vision region 20B can be appropriately obtained with expanded depth of focus. As one example, in the intraocular lens 1 illustrated in FIGS. 5 and 11, the central angle CAof the distant vision region 20A is 150 degrees, the central angle CB of the near vision region 20B is 100 degrees, and the central angle CC of the intermediate vision region 20C is 50 degrees.

In the intraocular lens 1 of this embodiment, the refractive power of the near vision region 20B is set to be greater than or equal to +2.5D and less than or equal to +4.0D, and the refractive power of the intermediate vision region 20C is set to be greater than or equal to +1.0D and less than or equal to +2.5D. In this case, both the multifocal effect by the plurality of segmented regions 20 and the depth of focus expansion effect can be appropriately obtained. In addition, it is more preferable that the refractive power of the near vision region 20B is set to be +3.0D or more and +3.5D or less, and the refractive power of the intermediate vision region 20C is set to be +1.5D or more and +2.0D or less. As one example, in the intraocular lens 1 illustrated in FIGS. 5 and 11, the refractive power of the distant vision region 20A is 0D, the refractive power of the near vision region 20B is +3.25D, and the refractive power of the intermediate vision region 20C is +2.0D.

It is also possible to form a toric surface on at least one of the front and rear surfaces of the lens portion 2 to correct astigmatism of the wearer. In this case, the intraocular lens 1 not only provides both distant vision and near vision, but also corrects astigmatism of the wearer. Note that the plurality of segmented regions 20 may be formed on the front surface of the lens surface (that is, the surface facing the front side of the eye (a cornea side) when the lens is placed in the wearer's eye). In this case, the shape of the rear surface of the lens surface tends to be smooth so that it is easier to prevent secondary cataract due to cells etc. entering between the rear surface of the lens surface and the posterior capsule of the eye from occurring. Furthermore, a plurality of segmented regions 20 and the toric surface may be formed together on the front surface of the lens surface. In this case, even when the toric surface is formed on the lens surface, occurrence of secondary cataract can be easily prevented. However, it is also possible to form at least one of the segmented region 20 and the toric surface on the rear surface of the lens surface. The surface on which the segmented region 20 is formed and the surface on which the toric surface is formed may be different.

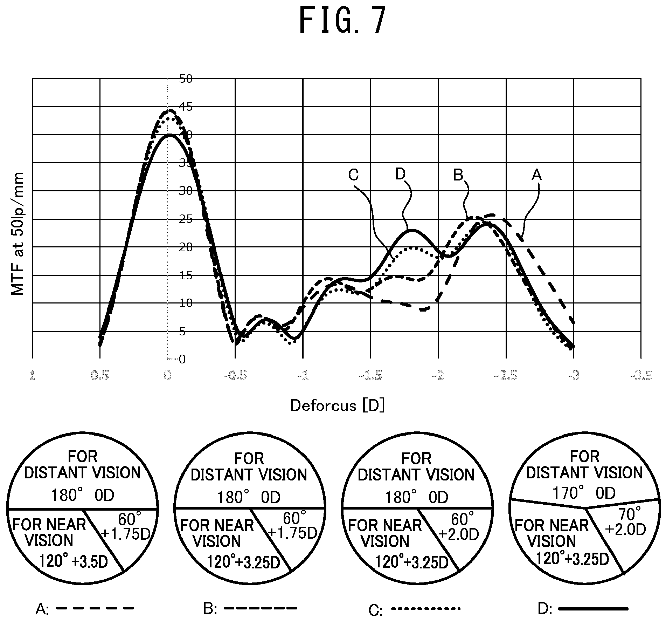

With reference to FIG. 7, the MTF characteristics will be described by changing the refractive power and central angle of each of the distant vision region 20A, the near vision region 20B, and the intermediate vision region 20C. MTF (Modulation Transfer Function) is an index indicating contrast. The graph of FIG. 7 shows an MTF curve at a spatial frequency of 50 lp/mm, with the horizontal axis representing the amount of defocus (i.e., the amount of focal shift, the amount of power deviation) and the vertical axis representing the MTF.

In the four intraocular lenses A, B, C, and D whose MTF curves are shown in FIG. 7, the reflective power and the central angle of each of the distant vision region 20A, the near vision region 20B, and the intermediate vision region 20C are changed within preferable conditions. Specifically, in the intraocular lens 1 of A, the central angle of the distant vision region 20A is 180 degrees and the refractive power is 0D, the central angle of the near vision region 20B is 120 degrees and the refractive power is +3.5D, and the central angle of the intermediate vision region 20C is 60 degrees and the refractive power is +1.75D. In the intraocular lens 1 of B, the central angle of the distant vision region 20A is 180 degrees and the refractive power is 0D, the central angle of the near vision region 20B is 120 degrees and the refractive power is +3.25D, and the central angle of the intermediate vision region 20C is 60 degrees and the refractive power is +1.75D. In the intraocular lens 1 of C, the central angle of the distant vision region 20A is 180 degrees and the refractive power is 0D, the central angle of the near vision region 20B is 120 degrees and the refractive power is +3.25D, and the central angle of the intermediate vision region 20C is 60 degrees and the refractive power is +2.0D. In the intraocular lens 1 of D, the central angle of the distant vision region 20A is 170 degrees and the refractive power is 0D, the central angle of the near vision region 20B is 120 degrees and the refractive power is +3.25D, and the central angle of the intermediate vision region 20C is 70 degrees and the refractive power is +2.0D. In each of the four intraocular lenses A, B, C, and D whose MTF curves are shown in FIG. 7, transition portions with central angles of 5 degrees are actually provided between each of the regions.

As shown in FIG. 7, in any of the four intraocular lenses A, B, C, and D, the MTF has a maximum value near the defocus amount 0D corresponding to far vision, and the MTF also has a maximum value in the range of -1.5D to -2.5D. Furthermore, in any intraocular lens 1, the depth of focus is appropriately expanded for both distant vision and near vision. From the above results, by setting the refractive power and central angle of each of the distant vision region 20A, near vision region 20B, and intermediate vision region 20C within the range of the preferable conditions as described above, both distant vision and near vision can be obtained with the expanded depth of focus. Furthermore, the MTF characteristics of the intraocular lens 1 can be adjusted by appropriately adjusting the refractive power and central angle of each of the distant vision region 20A, the near vision region 20B, and the intermediate vision region 20C.

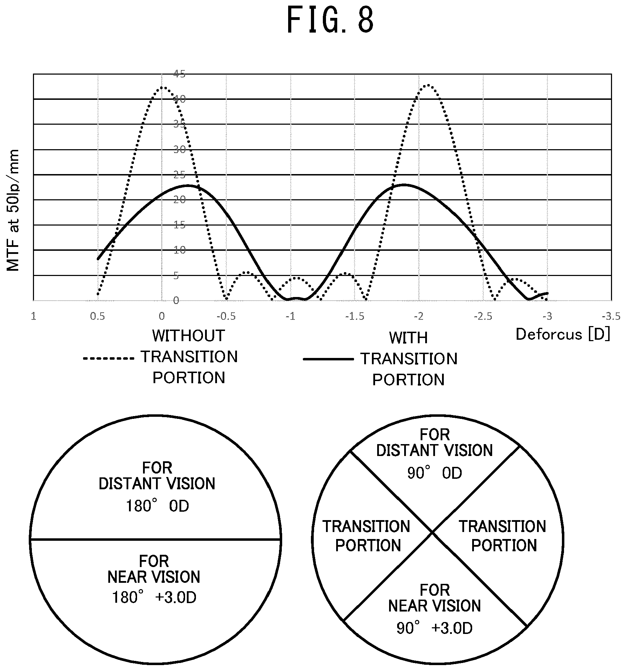

With reference to FIG. 8, the MTF characteristics will be described when no transition portion is formed between the distant vision region and the near vision region and when the transition portion is formed. The graph of FIG. 8 shows MTF curves at a spatial frequency of 50 lp/mm, with the horizontal axis representing the amount of defocus (the amount of focal shift, the amount of power deviation) and the vertical axis representing MTF, similar to the graph of FIG. 7.

FIG. 8 shows the MTF curve of an intraocular lens "without a transition portion" that does not form a transition portion between the distant and near vision regions and the MTF curve of an intraocular lens "with a transition portion" between the distant and near vision regions. Specifically, in the intraocular lens "without a transition portion", the central angle of the distant vision region is 180 degrees and the refractive power is 0D, and the central angle of the near vision region is 180 degrees and the refractive power is +3.0D. In the intraocular lens "with a transition portion", the central angle of the distant vision region is 90 degrees and the refractive power is 0D, and the central angle of the near vision region is 90 degrees and the refractive power is +3.0D. In addition, in an intraocular lens "with a transition portion", two transition portions each having a central angle of 90 degrees are formed between the distant vision region and the near vision region.

As shown in Fig. 8, in the intraocular lens "with a transition portion", the position of the maximum value of MTF corresponding to distant vision and the position of the maximum value of MTF corresponding to near vision are closer to each other than the positions of the maximum values of MTF for the intraocular lens "without a transition portion". Furthermore, the expanding effect of the depth of focus in the intraocular lens "with a transition portion" is improved as compared to the intraocular lens "without a transition portion". As described above, by forming the transition portion in the lens portion of the intraocular lens, the extended depth of focus (EDOF) effect of the multifocal intraocular lens can be appropriately obtained.

### (Cross-sectional area of the lens portion according to the first embodiment)

The cross-sectional area of the lens portion 2 of the intraocular lens 1 according to a first embodiment will be explained. As described with reference to FIG. 4, the intraocular lens in this embodiment is inserted into the patient's eye using the intraocular lens insertion instrument 100. Specifically, the intraocular lens 1 is moved forward in the pushing axis A along the passage of the intraocular lens insertion instrument 100 by the pushing member 310. When the intraocular lens 1 reaches the nozzle 180, the front supporter 3A comes into contact with the inner wall of the nozzle 180, which is tapered (that is, the passage area narrows toward the front side). Then, the direction of movement D of the lens 1 that is assumed in advance (see FIGS. 1 and 4) is substantially aligned with the pushing axis A of the intraocular lens insertion instrument 100. Thereafter, the lens portion 2 of the intraocular lens 1 is folded into a small size by contacting the tapered inner wall of the nozzle 180.

As shown in FIG. 5, it is assumed that a virtual cross-section in an arbitrary direction that passes through the geometric center O of the lens portion 2 and is perpendicular to the lens surface of the lens portion 2. A first cross-section S1, a second cross-section S2, and a third cross-section S3 shown in FIG. 5 are examples of a plurality of virtual cross-sections. As described above, in the intraocular lens 1 in this embodiment, the cross-sectional area of the lens portion 2 in the virtual cross-section changes depending on the direction in which the virtual cross-section is defined.

A virtual cross-section in a direction perpendicular to the pushing axis A (see FIGS. 2 and 4) of the intraocular lens insertion instrument 100 when the intraocular lens 1 is folded by the nozzle 180 of the intra lens insertion instrument 100 is defined as the first cross-section is S1. As described above, when the intraocular lens 1 reaches the nozzle 180, the assumed direction of movement D of the intraocular lens 1 is aligned with the pushing axis A. Therefore, the first cross-section S1 may also be defined as a virtual cross-section perpendicular to the direction of movement D of the intraocular lens 1 that is assumed in advance. In the intraocular lens 1 of the first embodiment, the cross-sectional area of the lens portion 2 in the first cross-section S1 is smaller than or equal to the cross-sectional areas in the virtual cross-sections defined by other directions. Therefore, when the intraocular lens 1 is moved out through the passage of the nozzle 180 at an appropriate angle with respect to the pushing axis A, the maximum cross-sectional area of the lens portion 2 in the direction perpendicular to the pushing axis A (i.e., the cross-sectional area of the first cross-section S1 when the cross-sectional area of the lens portion 2 in a folded state has the maximum value) decreases. Therefore, the intraocular lens 1 in the first embodiment can be folded into a small size within the passage of the nozzle 180.

The relationship between the first cross-section S1 and the configuration of the lens portion 2 will be further described. As described with reference to FIG. 6, in the lens portion 2, the radius of curvature of the portion with high refractive power is smaller than the radius of curvature of the portion with low refractive power. In other words, in the lens portion 2, the curve of the lens surface in the portion where the refractive power is large is steeper than the curve of the lens surface in the portion where the refractive power is small. Here, since it is optically undesirable to for a step at the geometric center O of the lens portion 2, the thickness of the lens portion at the geometric center O in the virtual cross-section is set to be the same regardless of the direction of the assumed virtual cross-section. (See FIG. 6). As a result, the thickness of the side surface (an edge portion) of the lens portion 2 in an area where the refractive power is large (i.e., an area where the curve of the lens surface is steep) is smaller than that of the lens portion where the refractive power is small (i.e., an area where the curve of the lens surface is gentle). In other words, in the lens portion 2, the thickness decreases as the refractive power increases.

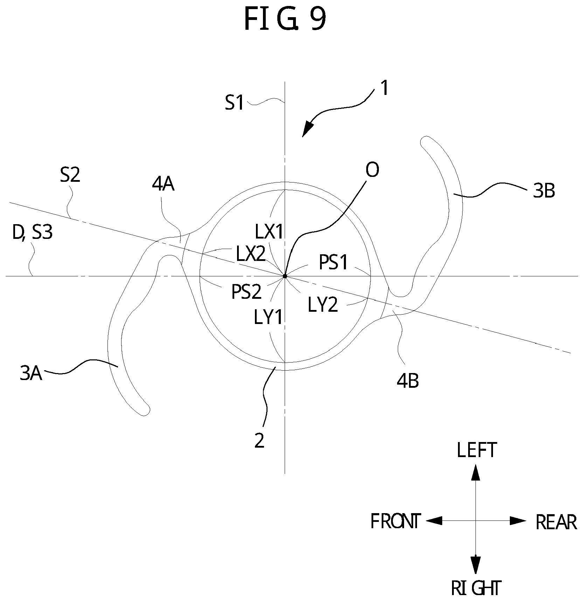

Here, in the lens portion 2, the total value of the refractive power on a line extending from the geometric center O along the virtual cross-section to one outer circumference and the refractive power on a line extending from the geometric center O along the same virtual plane to the other outer circumference is calculated for each of virtual cross-sections defined in arbitrary directions. For example, in the example shown in FIG. 9, the total value of the refractive power of a line LX1 extending from the geometric center O to one outer circumference along the first cross-section S1 (i.e., the refractive power 0D of the distant vision region 20A shown in FIG. 5) and the refractive power of a line LY1 extending from the geometric center O to the other outer circumference along the first cross-section S1 (i.e., the refractive power +3.25 of the near vision region 20B shown in FIG. 5) is +3.25D.

In the intraocular lens 1 according to the first embodiment, the lens portion 2 is designed such that the total value of the refractive powers on the two lines LX1, LY1 along the first cross-section S1 is equal to or more than the total value of the refractive powers on the two lines along the virtual cross-section in another direction. Therefore, since the thickness of the lens portion 2 decreases as the refractive power increases, the cross-sectional area of the lens portion 2 in the first cross-section S1 is equal to or smaller than the cross-sectional area of the lens portion 2 in the virtual cross-section in the other direction. As a result, since the cross-sectional area of the lens portion 2 in the first cross-section S1 is appropriately small, the intraocular lens 1 is easily folded into a small size within the passage of the nozzle 180.

Note that the configuration of the lens portion 2 in the first cross-section S1 may be changed. For example, the total value of the radius of curvature of the lens portion 2 on a line extending from the geometric center O to one outer circumference along the virtual cross-section (for example, the average value of the radius of curvature on the line, etc.) and the radius of curvature of the lens portion 2 on a line extending from the geometric center O to another circumference along the same virtual cross-section may be calculated for the virtual-cross-section in each arbitrary direction. The same goes for the following. ) and the total radius of curvature on a line extending from the geometric center O along the same virtual cross section to the other outer periphery is calculated for the virtual cross section in each arbitrary direction. In this case, the intraocular lens 1 may be designed such that the total value of the radii of curvature on the two lines LX1 and LY1 along the first cross-section S1 is equal to or smaller than the total value of the radii of curvature on two lines along a cross-section in another direction. Even in this case, the cross-sectional area of the lens portion 2 in the first cross-section S1 can be appropriately reduced.

The relationship between the base end portions 4 (4A, 4B) of the supporters 3 (3A, 3B) and the lens portion 2 will be explained. As shown in FIGS. 1, 5, and 9, the intraocular lens 1 in this embodiment includes a pair of supporters 3 (3A, 3B). The pair of supporters 3 extend outward from different locations at the side surface (i.e., an edge part) of the lens portion 2. It is preferable that the base end portion 4 of each of the pair of supporters 3 be connected to a thicker portion of the side surface of the lens portion 2 in order to stably support the lens portion 2 within the eye. Furthermore, if the proximal end portion 4 of the supporter 3 is connected to the thinner portion of the side surface of the lens portion 2, unintended deformation or damage to the lens portion 2 would occur when the intraocular lens 1 is inserted into the eye and the supporter 3 is bent.

Here, as shown in FIGS. 5 and 9, a virtual cross-section in a direction passing through the base end portions 4 of each of the pair of supporters 3 is defined as a second cross-section S2. In the intraocular lens 1 of the first embodiment, the cross-sectional area of the lens portion 2 in the second cross-section is greater than or equal to the cross-sectional area in the virtual cross-sections in other directions. As a result, the thicknesses of the portions of the side surface of the lens portion 2 to which the base end portions 4 of the pair of supporters 3 are connected increase. Therefore, since the rigidity of the proximal end portion 4 of the supporter 3 is secured, unintended deformation or breakage is less likely to occur, and thus the intraocular lens 1 is more easily installed in the eye. Further, since the amount of deformation of the pair of supporters 3 is equalized, the stability during inserting of the intraocular lens 1 into the eye is improved.

The relationship between the second cross-section S2 and the configuration of the lens portion 2 will be further explained. For example, in the example shown in FIG. 9, the total value of the refractive power of a line LX2 extending from the geometric center O to one outer circumference along the second cross-section S2 (i.e., the refractive power 0D of the distant vision region 20A shown in FIG. 5) and the refractive power of a line LY2 extending from the geometric center O to another outer circumference along the second cross-section S2 (i.e., the refractive power +2.0D of the intermediate vision region 20B shown in FIG. 5) is +2.0D. In the intraocular lens 1 according to the first embodiment, the lens portion 2 is designed such that the total value of the refractive powers on the two lines LX2, LY2 (see FIG. 9) along the second cross-section S2 is equal to or smaller than the total value of the refractive powers on the two lines along the virtual cross-section in another direction. Therefore, since the thickness of the lens portion 2 increases as the refractive power decreases, the cross-sectional area of the lens portion 2 in the second cross-section S2 is equal to or larger than the cross-sectional area of the lens portion 2 in the virtual cross-sections in the other directions. As a result, the thicknesses of the portions of the side surface of the lens portion 2 to which the base end portions 4 of the pair of supporters 3 are connected increase.

Note that the configuration of the lens portion 2 in the second cross-section S2 may be changed. For example, the intraocular lens 1 may be designed such that the total value of the radii of curvature on the two lines LX2 and LY2 along the second cross-section S2 is equal to or larger than the total value of the radii of curvature on two lines along a cross-section in another direction. Even in this case, the thicknesses of the portions of the side surface of the lens portion 2 to which the base end portions 4 of the pair of supporters 3 are connected increase.

As shown in FIG. 5, the plurality of segmented regions 20 (20A, 20B, 20C) are formed on at least one of the front surface and the rear surface of the lens portion of the intraocular lens 1 in the first embodiment. The plurality of segmented regions 20 extend radially outward from the center of the lens portion 2, and have different radii of curvature and thus have different refractive powers. In the intraocular lens 1 of the first embodiment, the plurality of segmented regions 20 (in the example shown in FIG. 5, the plurality of segmented regions 20 and transition portions 21) are arranged in the lens portion 2 such that the cross-sectional area of the lens portion 2 in the first cross-section S1 is equal to or smaller than the cross-sectional areas of the cross-sections in other directions. In addition, the plurality of segmented regions 20 (in the example shown in FIG. 5, the segmented regions 20 and the transition portions 21) are arranged in the lens portion 2 such that the cross-sectional area of the lens portion 2 in the second cross-section S2 is equal to or larger than the cross-sectional areas of the cross-sections in other directions. Therefore, unlike an intraocular lens in which a plurality of regions are concentrically arranged, even if the wearer's pupil becomes smaller, light passes through each of the plurality of segmented regions 20 and is focused on the retina. Furthermore, the plurality of segmented regions 20 have different radii of curvature and thus have different refractive powers. Therefore, the amount of light scattered in unintended directions is reduced as compared to a situation where a group of micro prisms or the like is formed in the segmented region. As a result, loss of light reaching the retina is less likely to occur, making it easier to obtain a better visual field. Further, the intraocular lens 1 is folded into a small size within the passage of the nozzle and inserted into the eye.

A mechanism for folding the vicinity of the proximal end portion 4B of the rear supporter 3B into a small size will be described. As explained in FIG. 4(e), when the intraocular lens 1 with the rear supporter 3B tacked is pushed out in the nozzle 180 by the pushing member 310, the load may increase when the vicinity of the proximal end 4B of the rear supporter 3B passes through the nozzle 180. The inventors found that this is because the volume of the pushing member 310 that is pushing out the intraocular tens 1 while being in contact with the rear supporter 3B is added to the increased volume of the intraocular lens 1 near the proximal end portion 4B due to the bent of the proximal end portion 4B of the rear supporter 3B. As a result, it would be difficult to bend the part of the intraocular lens 1 near the based end 4B of the rear supporter 3B into a small size.

Here, as shown in FIGS. 5 and 9, a virtual cross-section of the intraocular lens insertion instrument 100 in the direction along the pushing axis A (see FIGS. 2 and 4) is defined as a third cross-section S3. As described above, when the intraocular lens 1 reaches the nozzle 180, the assumed direction of movement D of the intraocular lens 1 is aligned with the pushing axis A. Therefore, the third cross-section S3 may also be expressed as a virtual cross-section in a direction along the direction of movement of the intraocular lens 1 that is assumed in advance. Further, as shown in FIGS. 5 and 9, in the third cross-section S3, a virtual partial cross-section extending rearward from the geometric center O along the pushing axis A (i.e., the direction of movement D) is defined as an on-axis rear cross-section PS1. In the third cross-section S3, a virtual partial cross-section extending forward from the geometric center O along the pushing axis A (i.e., the direction of movement D) is defined as an on-axis front cross-section PS2. In the intraocular lens 1 of the first embodiment, the cross-sectional area of the lens portion 2 in the on-axis rear cross-section PS1 is smaller than the cross-sectional area of the lens portion 2 in the on-axis front cross-section PS2. As a result, an increase in volume near the proximal end portion 4B of the rear supporter 3B adjacent to the pushing member 310 (see FIG. 4, etc.) is suppressed. Therefore, the intraocular lens 1 in the first embodiment is easily folded into a small size even at the vicinity of the proximal end 4B of the rear supporter 3B where the pushing member 310 is located. Therefore, in addition to the load generated when the geometric center O of the lens portion 2 passes through the nozzle 180, the load generated when the vicinity of the proximal end portion 4B of the rear supporter 3B passes through the nozzle 180 is also appropriately reduced.

As described above, when comparing each part of the lens portion 2, the cross-sectional area decreases as the refractive power increases (that is, as the radius of curvature decreases). Here, a virtual partial cross-section that is perpendicular to the lens surface of the lens portion 2 and extends in an arbitrary direction outward from the geometric center O of the lens portion 2 is assumed. In this case, the cross-sectional area of the lens portion 2 in the virtual partial cross-section decreases as the refractive power of the portion where the virtual partial cross-section is located increases (that is, the radius of curvature decreases). In the intraocular lens 2 of the first embodiment, the refractive power of the lens portion 2 in the on-axis rear cross-section PS1 is greater than the refractive power of the lens portion 2 in the on-axis front cross-section PS2. In other words, the radius of curvature of the lens portion 2 in the on-axis rear cross-section PS1 is smaller than the radius of curvature of the lens portion 2 in the on-axis front cross-section PS2. As a result, an increase in volume near the proximal end portion 4B of the rear supporter 3B adjacent to the pushing member 310 (see FIG. 4, etc.) is suppressed. Therefore, the intraocular lens 1 in the first embodiment is easily folded into a small size even at the vicinity of the proximal end 4B of the rear supporter 3B.

The relationship between the on-axis rear cross-section PS1/the on-axis front cross-section PS2 and the configuration of the lens portion 2 will be further described. As shown in FIG. 5, the plurality of segmented regions 20 (20A, 20B, 20C) are formed on at least one of the front surface and the rear surface of the lens portion 2 of the intraocular lens 1 in the first embodiment. The plurality of segmented regions 20 extend radially outward from the center of the lens portion 2, and have different radii of curvature and thus have different refractive powers. In the intraocular lens 1 of the first embodiment, a plurality of segmented regions 20 are arranged in the lens portion 2 (in the example shown in FIG. 5, a plurality of segmented regions20 and a plurality of transition portions 21) such that the refractive power of the segmented region through which the on-axis rear cross-section PS1 (in the example shown in FIG. 5, the intermediate vision region 20C) is greater than the refractive power of the segmented region through which the on-axis front section PS2 passes (in the example shown in FIG. 5, the distant vision region 20A) . Therefore, the intraocular lens 1 in the first embodiment has both the effect of providing a plurality of segmented regions 20 (the effect of providing a good visual field regardless of the size of the wearer's pupil) and the effect of folding the vicinity of the end portion 4B of the rear supporter 3B into a small size.

### (Modified example of the first embodiment)

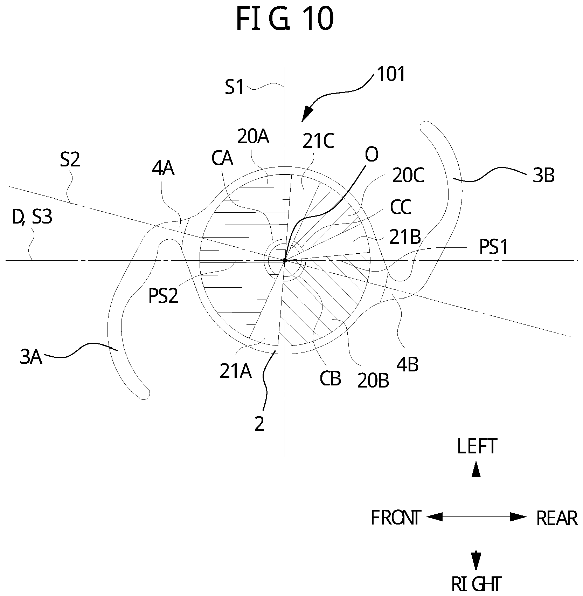

A modification to the first embodiment will be described with reference to FIG. 10. The intraocular lens 101 according to the modified example shown in FIG. 10 is different from the intraocular lens 1 shown in FIG. 5 as to the arrangement of a plurality of segmented regions 20 (21A, 21B, 21C) and a plurality of transition portions 21 (21A, 21B, 21C) that are formed in the lens portion 2.

In the intraocular lens 101 shown in FIG. 10, similarly to the intraocular lens 1 shown in FIG. 5, the cross-sectional area of the lens portion 2 in the first cross-section S1 is equal to or smaller than the cross-sectional areas in the virtual cross-sections in the other directions. Further, the total value of the refractive powers on the two lines LX1 and LY1 (not shown in FIG. 10) along the first cross-section S1 is greater than or equal to the total value of the refractive powers on the two lines along the virtual cross-section in the other direction. Furthermore, the total value of the radius of curvature on the two lines LX1 and LY1 along the first cross-section S1 is less than the total value of the radius of curvature on the two lines along the virtual cross-section in the other direction. Therefore, similar to the intraocular lens 1 shown in FIG.5, the intraocular lens 101 shown in FIG. 10 can also be folded into a smaller size near the geometric center O.

As described above, a virtual cross-section of the intraocular lens insertion instrument 100 in the direction along the pushing axis A (see FIGS. 2 and 4) is defined as the third cross-section S3. In the third cross-section S3, a virtual partial cross-section extending rearward from the geometric center O along the pushing axis A (i.e., the direction of movement D) is defined as an on-axis rear cross-section PS1. In the third cross-sections S3, a virtual partial cross-section extending forward from the geometric center O along the pushing axis A (i.e., the direction of movement D) is defined as an on-axis front cross-section PS2. In the intraocular lens 101 shown in FIG. 10, similarly to the intraocular lens 1 shown in FIG. 5, the cross-sectional area of the lens portion 2 in the on-axis rear cross-section PS1 is smaller than the cross-sectional area of the lens portion 2 in the on-axis front cross-section PS2. Further, the refractive power of the lens portion 2 in the on-axis rear cross-section PS1 is greater than the refractive power of the lens portion 2 in the on-axis front cross-section PS2. In other words, the radius of curvature of the lens portion 2 in the on-axis rear cross-section PS1 is smaller than the radius of curvature of the lens portion 2 in the on-axis front cross-section PS2. Furthermore, the plurality of segmented regions20 are arranged in the lens portion 2 such that the refractive power of the segmented region (i.e., the near vision region 20B in the example shown in FIG. 10) through which the on-axis rear section PS1 passes is greater than the refractive power of the segmented region (i.e., the distant vision region 20A in the example shown in FIG. 10) through which the on-axis front cross-section PS2. As a result, the intraocular lens 101 is easily folded into a small size near the proximal end 4B of the rear supporter 3B where the pushing member 310 is located.

In the intraocular lens 101 shown in FIG. 10, as described above, a virtual partial cross-section that is perpendicular to the lens surface of the lens portion 2 and extends in an arbitrary direction outward from the geometric center O of the lens portion 2 is also assumed. In the modified intraocular lens 101 shown in FIG. 10, the cross-sectional area of the on-axis rear cross-section PS1 is equal to or smaller than the cross-sectional area of the virtual partial cross-section in another direction. Further, the refractive power of the lens portion 2 in the on-axis rear cross-section PS1 is larger than the refractive power of the lens portion 2 in the virtual partial cross-section in another direction. In other words, the radius of curvature of the lens portion 2 in the on-axis rear cross-section PS1 is smaller than the radius of curvature of the lens portion in the virtual partial cross-section in the other direction. Therefore, an increase in volume of the proximal end portion 4B of the rear supporter 3B adjacent to the pushing member 310 (see FIG. 4, etc.) is further appropriately suppressed. Therefore, when the intraocular lens 101 is pushed out in the passage of the nozzle 180 by the pushing member 310, the in lens 101 can be more appropriately folded.

In the intraocular lens 101 shown in FIG. 10, the on-axis rear cross-section PS1 passes through the near vision region 20B having the largest refractive power among the plurality of segmented regions 20 (20A, 20B, 20C). As described above, among the plurality of segmented regions 20, the edge portion of the lens portion 2 has the smallest thickness in the near vision region 20B which has the largest refractive power. Therefore, in the intraocular lens 101 shown in FIG. 10, an increase in volume near the proximal end 4B of the rear supporter 3B that is close to the pushing member 310 is more appropriately suppressed.

### (Cross-sectional area of the lens portion according to the second embodiment)

The cross-sectional area of the lens portion 2 of the intraocular lens 1 according to the second embodiment will be described. As described with reference to FIG. 4, the intraocular lens in this embodiment is inserted into the patient's eye using the intraocular lens insertion instrument 100. Specifically, the intraocular lens 1 is moved forward in the pushing axis A along the passage of the intraocular lens insertion instrument 100 by the pushing member 310. When the intraocular lens 1 reaches the nozzle 180, the front supporter 3A comes into contact with the inner wall of the nozzle 180, which is tapered (that is, the passage area narrows toward the front side). Then, the direction of movement D of the lens 1 that is assumed in advance (see FIGS. 1 and 4) is substantially aligned with the pushing axis A of the intraocular lens insertion instrument 100. Thereafter, the lens portion 2 of the intraocular lens 1 is folded into a small size by contacting the tapered inner wall of the nozzle 180.

As explained in FIG. 4(e), when the intraocular lens 1 with the rear supporter 3B tacked is pushed out in the nozzle 180 by the pushing member 310, the load may increase when the vicinity of the proximal end 4B of the rear supporter 3B passes through the nozzle 180. The inventors found that this is because the volume of the pushing member 310 that is pushing out the intraocular tens 1 while being in contact with the rear supporter 3B is added to the increased volume of the intraocular lens 1 near the proximal end portion 4B due to the bent of the proximal end portion 4B of the rear supporter 3B. As a result, it would be difficult to bend the part of the intraocular lens 1 near the based end 4B of the rear supporter 3B into a small size. The mechanism of the intraocular lens 1 for bending the vicinity of the proximal end 4B of the rear supporter 3B into a small size will be described below.

First, a virtual cross-section will be described. The virtual cross-section is a cross-section of the lens portion 2 in an arbitrary direction that passes through the geometric center of the lens portion 2 and is perpendicular to the lens surface of the lens portion 2.

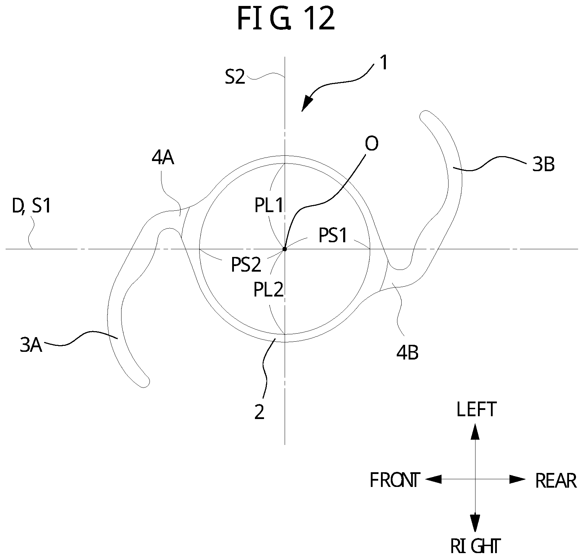

As shown in FIGS. 11 and 12, when an intraocular lens 1 is folded by a nozzle 180 of an intraocular lens insertion instrument 100, a virtual cross-section in a direction along a pushing axis A of the intraocular lens insertion instrument 100 (see FIGS. 2 and 4) is defined as an on-axis cross-section S1. As described above, when the intraocular lens 1 reaches the nozzle 180, the assumed direction of movement D of the intraocular lens 1 is aligned with the pushing axis A. Therefore, the on-axis cross-section S1 can also be expressed as a virtual cross-section in a direction passing through the geometric center O of the lens portion 2 along a direction of movement D of the intraocular lens 1 that is assumed in advance.

Next, a virtual partial cross-section will be explained. The virtual partial cross-section is a partial cross-section of the lens portion 2 that is perpendicular to the lens surface of the lens portion 2 and extends in an arbitrary direction outward from the geometric center of the lens portion 2.

As shown in FIGS. 11 and 12, when the intraocular lens 1 is folded by the nozzle 180 of the intraocular lens insertion instrument 100, a virtual partial cross-section extending rearward from the geometric center O of the lens portion 2 along the pushing axis A of the intraocular lens insertion instrument 100 (see FIG. 2 and FIG. 4) is defined as an on-axis rear cross-section PS1. The on-axis rear cross-section PS1 may also be expressed as a virtual partial cross-section extending from the geometric center O of the lens portion 2 in a direction opposite to the direction of movement D of the intraocular lens 1 (i.e., a rearward direction).

It should be noted that, when the intraocular lens 1 is folded by the nozzle 180 of the intraocular lens insertion instrument 100, a virtual partial cross-section extending forward from the geometric center O of the lens portion 2 along the pushing axis A of the intraocular lens insertion instrument 100 (see FIG. 2 and FIG. 4) is defined as an on-axis front cross-section PS2. The on-axis front cross-section PS2 may also be expressed as a virtual partial cross-section extending from the geometric center O of the lens portion 2 in the direction of movement D of the intraocular lens 1 (i.e., a forward direction). As shown in FIGS. 11 and 12, the on-axis cross-section S1 is formed of the on-axis rear cross-section PS1 and the on-axis front cross-section PS2.

In the intraocular lens 1 according to the second embodiment, the cross-sectional area of the lens portion 2 in the on-axis rear cross-section PS1 is smaller than or equal to the cross-sectional area in a virtual partial cross-section in any other direction. As a result, an increase in volume of a portion around the proximal end portion 4B of the rear supporter 3B that is close to the pushing member 310 is suppressed, and therefore the vicinity of the proximal end portion 4B of the rear supporter 3B is easily bent to a small size. Therefore, the intraocular lens 1 in the second embodiment is more easily folded into a smaller size when being pushed out by the pushing member 310 along the passage of the nozzle 180 of the intraocular lens insertion instrument 100.

As described above, in the lens portion 2, the radius of curvature of the portion with high refractive power is smaller than the radius of curvature of the portion with low refractive power. In other words, in the lens portion 2, the curve of the lens surface in the portion where the refractive power is large is steeper than the curve of the lens surface in the portion where the refractive power is small. Here, since it is optically undesirable to form a step at the geometric center O of the lens portion 2, the lens portion 2 at the geometric center O through which each virtual partial cross-section passes has the same thickness regardless of the direction of the assumed virtual partial cross-section. As a result, the thickness of the side surface (an edge portion) of the lens portion 2 in an area where the refractive power is large (i.e., an area where the curve of the lens surface is steep) is smaller than that of the lens portion where the refractive power is small (i.e., an area where the curve of the lens surface is gentle). Therefore, assuming a virtual partial cross-section in an arbitrary direction, the cross-sectional area of the lens portion 2 at the location where the virtual partial cross-section is positioned decreases as the refractive power of the virtual partial cross-section extending radially outward from the geometric center O increases.

In the intraocular lens 1 according to the second embodiment, the lens portion 2 is designed such that the refractive power of the lens portion 2 at the position of the on-axis rear cross-section PS1 is greater than or equal to the refractive power of the lens portion 2 at the position of the virtual partial cross-section in any other direction. Therefore, the cross-sectional area of the lens portion 2 at the position of the on-axis rear cross-section PS1 is appropriately reduced. As a result, an increase in volume of a portion around the proximal end portion 4B of the rear supporter 3B that is close to the pushing member 310 is suppressed, and therefore the vicinity of the proximal end portion 4B of the rear supporter 3B is easily bent to a small size.

In other words, in the intraocular lens 1 according to the second embodiment, the lens portion 2 is designed such that the radius of curvature of the lens portion 2 in the on-axis rear cross-section PS1 is equal to or less than the radius of curvature of the lens portion 2 in a virtual partial cross-section in any other direction. As a result, an increase in volume near the proximal end portion 4B of the rear supporter 3B adjacent to the pushing member 310 is suppressed.

The relationship between the on-axis rear cross-section PS1 and the configuration of the lens portion 2 will be further explained. As shown in FIG. 11, the plurality of segmented regions 20 (20A, 20B, 20C) are formed on at least one of the front surface and the rear surface of the lens portion 2 of the intraocular lens 1 in the first embodiment. The plurality of segmented regions 20 extend radially outward from the center of the lens portion 2, and have different radii of curvature and thus have different refractive powers. In the intraocular lens 1 according to the second embodiment, the on-axis rear cross-section PS1 passes through the segmented region having the largest refractive power among the plurality of segmented regions 20 (the near vision region 20B in the example shown in FIG. 11). The cross-sectional area of the lens portion 2 in the virtual partial cross-section passing through the near vision region 20B is smaller than that of the lens portion in the virtual partial cross-section passing through another area (the distant vision region 20A, the intermediate vision region 20C, and the transition portions 21A, 21B, 21C). Therefore, an increase in volume near the proximal end portion 4B of the rear supporter 3B adjacent to the pushing member 310 is suppressed. Therefore, the intraocular lens 1 in the second embodiment has both the effect of providing a plurality of segmented regions 20 (the effect of providing a good visual field regardless of the size of the wearer's pupil) and the effect of folding the vicinity of the end portion 4B of the rear supporter 3B into a small size.

Considering the load applied when the entire intraocular lens 1 passes through the passage of the nozzle 180, in addition to suppressing an increase in volume of the portion near the proximal end 4B of the rear supporter 3B close to the pushing member 310, It is also preferable to make the maximum value of the cross-sectional area of the lens portion 2 in a direction perpendicular to the pushing axis A (see FIGS. 2 and 4) as small as possible. Hereinafter, a mechanism for reducing the maximum value of the cross-sectional area of the lens portion 2 in the direction perpendicular to the pushing axis A will be described.

As shown in FIGS. 11 and 12, when the intraocular lens 1 is folded by the nozzle 180 of the intraocular lens insertion instrument 100, a pair of virtual partial cross-section that vertically extend from the geometric center O of the lens portion 2 in a direction perpendicular to the pushing axis A (see FIGS. 2 and 4) are defined as vertical partial cross-section PL1, PL2. In the second embodiment, a virtual partial cross-section extending from the geometric center O of the lens portion 2 in a left direction perpendicular to the pushing axis A is defined as the vertical partial cross-section PL1. Further, a virtual partial cross-section extending from the geometric center O of the lens portion 2 in a right direction perpendicular to the pushing axis A is defined as the vertical partial cross-section PL2. The pair of vertical partial cross-sections PL1 and PL2 may also be expressed as virtual partial cross-sections extending perpendicularly from the geometric center O of the lens portion 2 with respect to the direction of movement D of the intraocular lens 1 that is assumed in advance. As shown in FIGS. 11 and 12, the vertical section S2 is formed of the pair of vertical partial sections PL1 and PL2.

In the intraocular lens 1 according to the second embodiment, the lens portion 2 is designed such that the cross-sectional area of the lens portion 2 in at least one of the pair of vertical partial cross-sections PL1, PL2 (in the second embodiment, the vertical partial cross-section PL1) is equal to or smaller than the cross-section area of the lens portion 2 in a virtual partial cross-section in any other arbitrary direction. In other words, the cross-sectional area of the lens portion 2 in at least one of the pair of vertical partial cross-sections PL1 and PL2 matches the cross-sectional area of the lens portion 2 in the on-axis rear cross-section PS1. As a result, the maximum value of the cross-sectional area of the lens portion 2 in the direction perpendicular to the pushing axis A is also reduced. Therefore, in addition to the load applied when the vicinity of the proximal end portion 4B of the rear supporter 3B passes through the nozzle 180, the load applied when the geometric center O of the lens section 2 passes through the nozzle 180 is also appropriately reduced.

As described above, assuming a virtual partial cross-section in an arbitrary direction, the cross-sectional area of the lens portion 2 at a portion where the virtual partial cross-section is located decreases as the refractive power of the virtual partial cross-section increases. In the intraocular lens 1 according to the second embodiment, the intraocular lens 1 is designed such that the refractive power of the lens portion 2 at the location where at least one of the pair of vertical partial cross-sections PL1 and PL2 is located is equal to or greater than the refractive power of the lens portion 2 at the location where a virtual partial cross-section in any other arbitrary direction. As a result, the maximum value of the cross-sectional area of the lens portion 2 in the direction perpendicular to the pushing axis A is appropriately reduced.

In other words, in the intraocular lens 1 according to the second embodiment, the intraocular lens 1 is designed such that the radius of curvature of the lens portion 2 in at least one of the pair of vertical partial cross-sections PL1, PL2 is equal to or smaller than the radius of curvature of the lens portion 2 in a virtual partial cross-section in any other arbitrary direction. As a result, the maximum value of the cross-sectional area of the lens portion 2 in the direction perpendicular to the pushing axis A is appropriately reduced.

The relationship between the pair of vertical partial cross-sections PL1 and PL2 and the configuration of the lens portion 2 will be further described. As shown in FIG. 11, a plurality of segmented regions 20 (20A, 20B, 20C) are formed in the lens portion 2 of the intraocular lens 1 in the second embodiment. In the intraocular lens 1 of the second embodiment, at least one of the pair of vertical partial cross-sections PL1 and PL2 passes through the segmented region having the maximum refractive power among the plurality of segmented regions (in the example shown in FIG. 11, the near vision region 20B). The cross-sectional area of the lens portion 2 in the virtual partial cross-section passing through the near vision region 20B is smaller than that of the lens portion in the virtual partial cross-section passing through another area (the distant vision region 20A, the intermediate vision region 20C, and the transition portions 21A, 21B, 21C). Therefore, the maximum value of the cross-sectional area of the lens portion 2 in a direction perpendicular to the pushing axis A is appropriately reduced by at least one of the pair of vertical partial cross-sections PL1 and PL2 passing through the near vision region 20B.

The techniques disclosed in the above embodiments are merely examples. Therefore, it is also possible to modify the techniques exemplified in the above embodiments. First, it is also possible to employ only some of the techniques illustrated in the above embodiments for the intraocular lens. For example, it is also possible to adopt only a technique of forming three or more segmented regions 20 with mutually different radii of curvature in the lens portion 2 without using the technique of reducing the cross-sectional area of the lens portion 2 in the first cross-section S1. In this case, the intraocular lens may be inserted into the eye without using the intraocular lens insertion instrument 100. Furthermore, three or more segmented regions may be formed in an intraocular lens without a supporter. Even when three or more segmented regions are formed in an intraocular lens with supporters, the number, shape, etc. of the supporters are not necessarily limited to those exemplified in the above embodiments.

Further, in the first embodiment, the technique of reducing the cross-sectional area of the lens portion 2 in the first cross-section S1 may be only used without using the technique of forming three or more segmented regions 20 in the lens portion 2. For example, the technique of reducing the cross-sectional area of the lens portion 2 in the first cross-section S1 may be applied to a toric intraocular lens for correcting astigmatism of a wearer. In this case, the toric intraocular lens may be designed such that the position of the first cross-section is aligned with the strong principal meridian of the lens portion. Also in this case, the cross-sectional area of the lens portion in the first cross-section is appropriately reduced.

In the second embodiment, it is possible to avoid using the technique for making the cross-sectional area of the lens portion 2 in at least one of the pair of vertical partial cross-sections PL1 and PL2 equal to or smaller than the cross-sectional area of the lens portion 2 in a virtual partial cross-section in any other direction. Even in this case, by making the cross-sectional area of the lens portion 2 in the on-axis rear cross-section PS1 equal to or less than the cross-sectional area in the virtual partial cross-section in any other direction, the vicinity of the proximal end 4B of the rear supporter 3B can be bent with a small size.

In the second embodiment, the intraocular lens 1 may be designed such that the on-axis rear cross-section PS1 passes through the vicinity of the center of the segmented region having the largest refractive power (the near vision region 20B in the second embodiment) among the plurality of segmented regions 20. In this case, the vicinity of the proximal end portion 4B of the rear supporter 3B is likely to be bent even smaller. Note that the range near the center of the near vision region 20B through which the on-axis rear cross-section PS1 passes may be set as appropriate. For example, it is assumed that the central angle of the fan-shaped near vision region 20B is K degrees. Further, a line passing through the geometric center O and equally dividing the fan-shaped near vision region 20B into two is defined as a center line. In this case, the on-axis rear cross-section PS1 may be set within a range of angle of 0.2K or less of the on-axis rear cross-section PS1 with respect to the center line, more preferably a range of angle of 0.1K degrees or less. In this case, the vicinity of the proximal end portion 4B of the rear supporter 3B is likely to be bent even smaller.

The technique illustrated in the second embodiment may also be applied to an intraocular lens in which a plurality of segmented regions 20 are not formed (for example, a toric intraocular lens). Further, both the plurality of segmented regions 20 and a toric surface for correcting astigmatism in the wearer may be formed in the lens portion 2.

Further, in the first and second embodiments, by the front supporter 3A of the intraocular lens 1 coming into contact with the tapered inner wall of the nozzle 180, the direction of movement D of the intraocular lens 1, which is assumed in advance, is substantially aligned with the pushing axis A of the intraocular lens insertion instrument 100. However, it is also possible to change the method of causing the direction of movement of the intraocular lens 1 to be substantially aligned with the pushing axis A of the intraocular lens insertion instrument 100. For example, a guide or the like for causing the direction of movement D of the intraocular lens 1 to be substantially aligned with the pushing axis A may be provided separately.

## Claims

1. An intraocular lens that is deformable and is inserted into a patient's eye from an insertion port formed at a front end of an intraocular lens insertion instrument after passing through an internal passage of the intraocular lens insertion instrument, the intraocular lens insertion instrument including:
a pushing member that is configured to push the intraocular lens forward by moving forward in the internal passage along a pushing axis that is an axis of the internal passage; and
a nozzle that includes the insertion port at the front end and defines therein the internal passage that is tapered toward a front side of the nozzle, the intraocular lens being discharged from the nozzle through the insertion port after the intraocular lens was folded while being pushed forward in the internal passage by the pushing member, the intraocular lens comprising:
a lens portion having a disc-shape, wherein
a cross-section of the lens portion defined by an arbitrary direction that passes through a geometric center of the lens portion and is perpendicular to a lens surface of the lens portion is defined as a virtual cross-section,
a cross-sectional area of the lens portion in the virtual cross-section changes depending on the arbitrary direction that defines the virtual cross-section, and
when the intraocular lens is folded by the nozzle, a cross-sectional area of the lens portion in a first cross-section, which is the virtual cross-section defined by a direction perpendicular to the pushing axis, is smaller than or equal to a cross-sectional area in the virtual cross-section that is defined by another direction.

2. The intraocular lens according to claim 1, wherein
when a total value of a refractive power on a line extending from the geometric center along the virtual cross-section to one outer circumference and a refractive power on a line extending from the geometric center along the virtual cross-section to the other outer circumference is calculated for each of virtual cross-sections defined by arbitrary directions,
a total value of refractive powers on lines along the first cross-section is greater than or equal to a total value of refractive powers on lines along the virtual cross-section defined by another direction.

3. The intraocular lens according to claim 1, further comprising:
a pair of supporters that extend outward from different parts of side surfaces of the lens portion to support the lens portion within the patient's eye, wherein
a cross-sectional area of the lens portion in a second cross-section, which is the virtual cross-section defined by a direction that passes through proximal end portions of the pair of supporters is equal to or larger than a cross-sectional area of the virtual cross-section defined by another direction.

4. The intraocular lens according to claim 3, wherein
when a total value of a refractive power on a line extending from the geometric center along the virtual cross-section to one outer circumference and a refractive power on a line extending from the geometric center along the virtual cross-section to the other outer circumference is calculated for each of virtual cross-sections defined by arbitrary directions,
a total value of refractive powers on lines along the second cross-section is less than or equal to a total value of refractive powers on lines along the virtual cross-section defined by another direction.

5. The intraocular lens according to claim 1, wherein
a plurality of segmented regions are formed on at least one of a front surface and a rear surface of the lens portion, the segmented regions extending radially outward from a center portion of the lens portion and having different refractive powers due to mutually different radii of curvature, and
the plurality of segmented regions are arranged in the lens portion such that a cross-sectional area of the lens portion in the first cross-section is smaller than or equal to a cross-sectional area in the virtual cross-section defined by another direction.

6. The intraocular lens according to claim 1, further comprising:
one or more supporters extending outward from the lens portion to have a curved shape, wherein
the pushing member is configured to push out, by moving forward in the internal passage along the pushing axis, the intraocular lens forward while a rear supporter, which is the one or more supporters extending rearward from the lens portion, is being folded onto the lens portion with the pushing member in contact with the rear supporter,
the virtual cross-section defined by a direction along the pushing axis is defined as a third cross-section,
the third cross-section includes an on-axis rear cross-section that extends rearward from the geometric center along the pushing axis and an on-axis front cross-section that extends forward from the geometric center along the pushing axis, and
a cross-sectional area of the on-axis rear cross-section is smaller than a cross-sectional area of the on-axis front cross-section.

7. The intraocular lens according to claim 6, wherein
a plurality of segmented regions are formed on at least one of a front surface and a rear surface of the lens portion, the segmented regions extending radially outward from a center portion of the lens portion and having different refractive powers due to mutually different radii of curvature, and
a refractive power of a first one of the segmented regions through which the on-axis rear cross-section passes is greater than a refractive power of a second one of the segmented regions through which the on-axis front cross-section passes.

8. The intraocular lens according to claim 6, wherein
a cross-section of the lens portion that is perpendicular to a lens surface of the lens portion and extends in an arbitrary direction outward from the geometric center of the lens portion is defined as a virtual partial cross-section, and
a cross-sectional area of the on-axis rear cross-section is smaller than or equal to a cross-sectional area of the virtual partial cross-section defined by another direction.

9. The intraocular lens according to claim 6, wherein
a plurality of segmented regions are formed on at least one of a front surface and a rear surface of the lens portion, the segmented regions extending radially outward from a center portion of the lens portion and having different refractive powers due to mutually different radii of curvature, and
the on-axis rear cross-section passes through one of the segmented regions having a largest refractive power.

10. An intraocular lens insertion instrument that is configured to insert an intraocular lens, which has been filled in advance and is deformable, into a patient's eye through an insertion port formed at a front end of the intraocular lens insertion instrument after passing through an internal passage of the intraocular lens insertion instrument, the instrument comprising:
a pushing member that is configured to push the intraocular lens forward by moving forward in the internal passage along a pushing axis that is an axis of the internal passage; and
a nozzle that includes the insertion port at the front end and defines therein the internal passage that is tapered toward a front side of the nozzle, the intraocular lens being discharged from the nozzle through the insertion port after the intraocular lens was folded while being pushed forward in the internal passage by the pushing member, wherein
the intraocular lens includes a lens portion having a disc-shape,
a cross-section of the lens portion defined by an arbitrary direction that passes through a geometric center of the lens portion and is perpendicular to a lens surface of the lens portion is defined as a virtual cross-section,
a cross-sectional area of the lens portion in the virtual cross-section changes depending on the arbitrary direction that defines the virtual cross-section, and
when the intraocular lens is folded by the nozzle, a cross-sectional area of the lens portion in a first cross-section, which is the virtual cross-section defined by a direction perpendicular to the pushing axis, is smaller than or equal to a cross-sectional area in the virtual cross-section defined by another direction.

11. An intraocular lens that is deformable and is inserted into a patient's eye from an insertion port formed at a front end of an intraocular lens insertion instrument after passing through an internal passage of the intraocular lens insertion instrument, the intraocular lens comprising:
a lens portion that has a disc-shape; and
one or more supporters that outwardly extend from the lens portion to have a curved shape, wherein
the intraocular lens insertion instrument includes:
a pushing member that is configured to push out, by moving forward in the internal passage along a pushing axis that is an axis of the internal passage, the intraocular lens forward while a rear supporter, which is the one or more supporters extending rearward from the lens portion, is being folded onto the lens portion by the pushing member in contact with the rear supporter; and
a nozzle that includes the insertion port at the front end and defines therein the internal passage that is tapered toward a front side of the nozzle, the intraocular lens being discharged from the nozzle through the insertion port after the intraocular lens was folded while being pushed forward in the internal passage by the pushing member,
a cross-section that is perpendicular to a lens surface of the lens portion and extends in an arbitrary direction outward from the geometric center of the lens portion is defined as a virtual partial cross-section,
a cross-sectional area of the lens portion in the virtual partial cross-section changes depending on the arbitrary direction that defines the virtual partial cross-section, and
when the intraocular lens is folded by the nozzle, a cross-sectional area of an on-axis rear cross-section, which is the virtual partial cross-section extending rearward from the geometric center along the pushing axis, is smaller than or equal to a cross-sectional area of the virtual partial cross-section defined by another direction.

12. The intraocular lens according to claim 11, wherein
a refractive power of the lens portion in the on-axis rear cross-section is greater than or equal to a refractive power of the lens portion in the virtual partial cross-section defined by another direction.

13. The intraocular lens according to claim 11, wherein
a pair of virtual partial cross-sections that extend from the geometric center in a direction perpendicular to the pushing axis are defined as a pair of vertical partial cross-sections, and
at least one of the pair of vertical partial cross-sections has a cross-sectional area that is smaller than or equal to that of the virtual partial cross-section defined by another direction.

14. The intraocular lens according to claim 11, wherein
a plurality of segmented regions are formed on at least one of a front surface and a rear surface of the lens portion, the segmented regions extending radially outward from a center portion of the lens portion and having different refractive powers due to mutually different radii of curvature, and
the on-axis rear cross-section passes through one of the segmented regions having a largest refractive power.

15. An intraocular lens insertion instrument that is configured to insert an intraocular lens, which has been filled in advance and is deformable, into a patient's eye through an insertion port formed at a front end of the intraocular lens insertion instrument after passing through an internal passage of the intraocular lens insertion instrument, the intraocular lens including a lens portion having a disc-shape and one or more supporters that outwardly extend from the lens portion to have a curved shape, the instrument comprising:
a pushing member that is configured to push out, by moving forward in the internal passage along a pushing axis that is an axis of the internal passage, the intraocular lens forward while a rear supporter, which is the one or more supporters extending rearward from the lens portion, is being folded onto the lens portion by the pushing member in contact the rear supporter; and
a nozzle that includes the insertion port at the front end and defines therein the internal passage that is tapered toward a front side of the nozzle, the intraocular lens being discharged from the nozzle through the insertion port after the intraocular lens was folded while being pushed forward in the internal passage by the pushing member,
a cross-section that is perpendicular to a lens surface of the lens portion and extends in an arbitrary direction outward from the geometric center of the lens portion is defined as a virtual partial cross-section,
a cross-sectional area of the lens portion in the virtual partial cross-section changes depending on the arbitrary direction that defines the virtual partial cross-section, and
when the intraocular lens is folded by the nozzle, a cross-sectional area of an on-axis rear cross-section, which is the virtual partial cross-section extending rearward from the geometric center along the pushing axis, is smaller than or equal to a cross-sectional area of the virtual partial cross-section defined by another direction.
